# EUROPEAN PATENT SPECIFICATION

(11) **EP 3 045 114 B1**
(45) Date of publication and mention of the grant of the patent: **03.04.2019**
(21) Application number: 16150630.8
(22) Date of filing: 08.01.2016
(51) Int. Cl.: A61B 6/02, A61B 6/00

(54) **CONTROL APPARATUS FOR CONTROLLING TOMOSYNTHESIS IMAGING, RADIOGRAPHING APPARATUS, CONTROL SYSTEM, CONTROL METHOD, AND RECORDING MEDIUM**
STEUERUNGSVORRICHTUNG ZUR STEUERUNG VON TOMOSYNTHESEBILDGEBUNG, RÖNTGENVORRICHTUNG, STEUERUNGSSYSTEM, STEUERUNGSVERFAHREN UND AUFZEICHNUNGSMEDIUM
APPAREIL DE COMMANDE POUR IMAGERIE PAR TOMOSYNTHÈSE, DISPOSITIF DE RADIOGRAPHIE, SYSTÈME DE COMMANDE, PROCÉDÉ DE COMMANDE ET SUPPORT D'ENREGISTREMENT

(30) Priority: 15.01.2015 JP 2015005926
(43) Date of publication of application: 20.07.2016
(73) Proprietor: Canon Kabushiki Kaisha, Tokyo 146-8501 (JP)
(72) Inventor: FUKUDA, Yuta, Ohta-ku, Tokyo 146-8501 (JP)
(74) Representative: Houle, Timothy James

(56) References cited:
- WO-A1-2014/145452
- JP-A- 2015 000 322
- US-A1- 2009 123 052

## Description

### BACKGROUND OF THE INVENTION

### Field of the Invention

The present invention relates to control performed by an apparatus and a system for controlling tomosynthesis imaging.

### Description of the Related Art

In tomosynthesis imaging, for example, while moving an X-ray generator, the apparatus irradiates a subject with X-rays emitted from different angles, and detects X-rays that have penetrated the subject via an X-ray detector to successively capture projection images of a plurality of frames having different imaging angles. The apparatus applies reconstruction processing such as the Filtered Back Projection (FBP) method to the projection images of a plurality of frames by using information of the positional relationship between the X-ray generator and the X-ray detector. Japanese Patent Application Laid-Open No. 2000-46760 discusses a technique for generating a tomographic image for a predetermined coronal cross section of a subject (a cross section parallel to the detection surface of an X-ray detector), and generating an oblique cross section intersecting with the detection surface of the X-ray detector.

Even in a case where a specific tomographic image such as an oblique image is used for diagnosis, a vast amount of data including projection image data and three-dimensional volume data is required to acquire the oblique cross section. In this case, there has been a problem of an increase in the load on communication between medical apparatuses, particularly on the communication from an imaging apparatus to a workstation and a Picture Archiving and Communication System (PACS), i.e., a medical image management server.

US 2009/0123052 describes a 2D mammogram image is synthesized from at least one of tomosynthesis projection images and/or the tomosynthesis reconstructed image data. In a simplest form, the mammogram may be synthesized by selecting one of the tomosynthesis projection images for display as a synthesized mammogram. Other methods of synthesizing a mammogram include re-projecting and filtering projection data and/or reconstructed data. The synthesized mammogram is displayed together with at least a portion of the reconstructed data to aid in review of the reconstructed data.

WO 2014/145452 describes a system and methods of display and viewing of three dimensional (3D) tomographic data acquired in tomosynthesis or tomography. A set of projection data is acquired with an image acquisition system and used to reconstruct enhanced 3D volume renderings that are viewed with motion, advanced image processing or stereotactically to assist in medical diagnosis.

### SUMMARY OF THE INVENTION

According to a first aspect of the present invention, there is provided an apparatus for controlling tomosynthesis imaging as specified in claims 1 to 11. According to a second aspect of the present invention, there is provided an image management apparatus as specified in clam 12. According to a third aspect of the present invention, there is provided a method for controlling tomosynthesis imaging as specified in clam 13. According to a fourth aspect of the present invention, there is provided a computer-readable recording medium that records a program for controlling tomosynthesis imaging as specified in clam 14.

Further features of the present invention will become apparent from the following description of embodiments with reference to the attached drawings. Each of the embodiments of the present invention described below can be implemented solely or as a combination of a plurality of the embodiments or features thereof where necessary or where the combination of elements or features from individual embodiments in a single embodiment is beneficial.

### BRIEF DESCRIPTION OF THE DRAWINGS

Fig. 1 is a block diagram illustrating a configuration of an X-ray radiography system according to an embodiment of the present invention.
Fig. 2 is a system configuration diagram when tomosynthesis imaging is performed according to an embodiment of the present invention.
Fig. 3 is a diagram illustrating position information acquired when tomosynthesis imaging is performed.
Fig. 4 is a block diagram illustrating a configuration of an imaging control unit according to an embodiment of the present invention.
Fig. 5 is a block diagram illustrating a hardware configuration of the imaging control unit according to an embodiment of the present invention.
Fig. 6 is a flowchart illustrating a processing flow from a start to an end of an examination for tomosynthesis imaging according to an embodiment of the present invention.
Fig. 7 is a flowchart illustrating a processing flow for displaying an oblique image according to an embodiment of the present invention.
Fig. 8 is a diagram illustrating a patient information input screen according to an embodiment of the present invention.
Fig. 9 is a diagram illustrating an imaging technique selection screen according to an embodiment of the present invention.
Fig. 10 is a diagram illustrating an imaging screen according to an embodiment of the present invention.
Fig. 11 is a diagram illustrating a coronal cross section display screen in a reconstruction screen according to an embodiment of the present invention.
Fig. 12 is a diagram illustrating an oblique cross section display screen in the reconstruction screen according to an embodiment of the present invention.
Fig. 13 is a diagram illustrating an example of a display when a button for shifting to the oblique cross section display screen in the coronal cross section display screen in the reconstruction screen according to an embodiment of the present invention.
Fig. 14 is a diagram illustrating an example of a display of a first threshold value region and a second threshold value region of an oblique angle in the coronal cross section display screen in the reconstruction screen according to an embodiment of the present invention.
Fig. 15 is a diagram illustrating an example of a display when an operator clicks inside of the first threshold value region and outside of the second threshold value region in the coronal cross section display screen in the reconstruction screen according to an embodiment of the present invention.
Fig. 16 is a diagram illustrating an example of a display when an operator clicks inside of the second threshold value region in the coronal cross section display screen in the reconstruction screen according to an embodiment of the present invention.
Fig. 17 is a diagram illustrating an example of a display when an operator specifies two target points and presses the button for shifting to the oblique cross section display screen in the coronal cross section display screen in the reconstruction screen according to an embodiment of the present invention.
Fig. 18 is a diagram illustrating an example of a display when two target points are specified and a warning message indicating that a generated oblique cross section does not pass through an isocenter line in the coronal cross section display screen in the reconstruction screen according to an embodiment of the present invention.
Fig. 19 illustrates an example of a display when the operator specifies three target points and presses the button for shifting to the oblique cross section display screen in the coronal cross section display screen in the reconstruction screen according to the embodiment of the present invention.
Fig. 20 is a diagram illustrating an example of a display when three target points are specified and a warning message indicating that a generated oblique cross section is close to a sagittal cross section in the coronal cross section display screen in the reconstruction screen according to an embodiment of the present invention.
Fig. 21 is a flowchart illustrating a processing flow according to another embodiment.
Fig. 22 is a diagram illustrating a positional relation between a coronal cross section and an oblique cross section according to an embodiment of the present invention.
Fig. 23 is a diagram illustrating a graphical user interface (GUI) for specifying a region in a tomographic image according to an embodiment of the present invention.
Fig. 24 is a diagram illustrating a positional relation between a specified region, a coronal cross section, and an oblique cross section.

### DESCRIPTION OF THE EMBODIMENTS

An X-ray imaging system capable of performing tomosynthesis imaging according to an embodiment of the present invention will be described below with reference to the accompanying drawings. The tomosynthesis imaging is an imaging method for capturing a plurality of projection images for acquiring a tomographic image of a subject by performing X-ray imaging on the subject from a plurality of different directions.

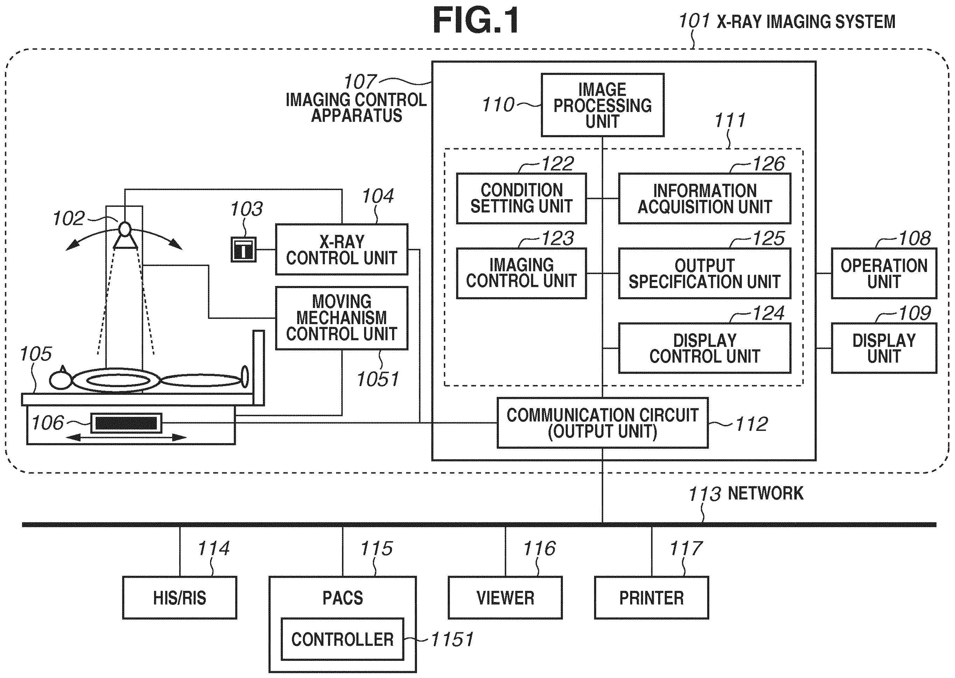

A configuration and operations of the X-ray imaging system as an example of a radiographing system according to the present embodiment will be described below with reference to Fig. 1. The X-ray imaging system may be referred to as an X-ray imaging apparatus.

An X-ray imaging system 101 includes an X-ray generation apparatus 102, an X-ray detector 106, and an imaging control apparatus 107, and is capable of performing tomosynthesis imaging. The imaging control apparatus 107 is a control apparatus for controlling tomosynthesis imaging. The imaging control apparatus 107 includes a communication circuit 112 for communicating with the X-ray detector 106, an image processing unit 110 including a Graphic Processing Unit (GNU), and a control unit 111 including at least one central processing unit. The control unit 111 is a control circuit for controlling the X-ray detector 106, image processing by the GNU, and the contents to be displayed on a display unit 109. The communication circuit 112 functions as an acquisition unit for acquiring from the X-ray detector 106 a plurality of projection images of the subject acquired through tomosynthesis imaging.

The X-ray generation apparatus 102 includes a target for generating X-rays when electrons collide therewith, and a diaphragm for shaping the generated X-ray flux, and irradiates the subject with X-rays shaped into a cone beam or a quadrangular beam. The X-ray generation apparatus 102 is fixed to an imaging table 105, and irradiates the subject with X-rays while being moved by a moving mechanism controlled by a moving mechanism control unit 1051. With X-ray irradiation, the moving mechanism control unit 1051 transmits an imaging implementation condition such as the tube voltage and the tube current, and position information such as the imaging angle and the X-ray source moving distance to an X-ray control unit 104. The X-ray control unit 104 is connected with the X-ray generation apparatus 102, an X-ray irradiation switch 103, and the imaging control apparatus 107. The X-ray control unit 104 controls the start and the end of X-ray irradiation and transmits the imaging implementation condition and the position information.

The X-ray generation apparatus 102 may receive a default imaging condition and default position information from the X-ray control unit 104, and perform imaging preparation processing. The X-ray irradiation switch 103, which may be connected to the X-ray generation apparatus 102, transmits an irradiation start notification and an irradiation end notification to the X-ray control unit 104. When an operator presses the X-ray irradiation switch 103, the X-ray irradiation switch 103 transmits an irradiation start notification. When the operator releases the X-ray irradiation switch 103, the X-ray irradiation switch 103 transmits an irradiation end notification. The X-ray control unit 104 may receive the default imaging condition and the default position information from the imaging control apparatus 107, and notify the X-ray generation apparatus 102 of the default imaging condition and the default position information.

The X-ray detector 106 is an image capturing unit for detecting X-rays that have penetrated the subject, and for converting the X-rays into X-ray image data. The X-ray detector 106 includes a scintillator for converting X-rays into visible light, and a sensor array in which a plurality of pixels are two-dimensionally arranged for converting visible light into an electrical signal. The X-ray detector 106, which is connected with the imaging control apparatus 107 via a cable or a wireless method, changes the state of power supply to the sensor array according to a control from the imaging control apparatus 107. The X-ray detector 106 transmits the X-ray image data to the imaging control apparatus 107. For example, the communication circuit 112 transmits an X-ray irradiation preparation request and an X-ray irradiation preparation cancellation request to the X-ray control unit 104 and the X-ray detector 106 via a communication interface (I/F). The communication circuit 112 further receives the X-ray image data, the imaging information, and the position information from the X-ray control unit 104 and the X-ray detector 106.

The X-ray detector 106 may transmit the converted X-ray image data together with imaging implementation information such as a reading area and a binning size to the imaging control apparatus 107. The X-ray detector 106 may also transmit the position information such as an X-ray detector moving distance together with the X-ray image data to the imaging control apparatus 107. The X-ray detector 106 may further perform imaging preparation processing upon reception of the default position information from the imaging control apparatus 107.

An operation unit 108 receives input operations for setting a user-requested imaging condition and an image processing condition such as a reconstruction condition, confirming projection images and reconstructed images, and transmitting images to an external apparatus. The operation unit 108 includes a keyboard, a mouse, a touch panel integrally formed, for example, with the display unit 109, or a combination of these devices.

The display unit 109 may display a user interface of control software for X-ray imaging. The display unit 109 may be an independent monitor or a monitor built in the X-ray imaging apparatus. A plurality of monitors for displaying a captured image may be connected to one imaging control apparatus 107. A captured image and a past image may be previewed on respectively different monitors. In this case, the display unit 109 determines which image is to be displayed on which monitor according to a notification from the imaging control apparatus 107, and displays the target image on the monitor determined to be used.

In addition, the X-ray imaging system 101 may be connected with a Hospital Information System (HIS)/Radiology Information System (RIS) 114, a Picture Archiving And Communication System (PACS) 115, a viewer 116, and a printer 117 via the imaging control apparatus 107 and a network 113. The HIS/RIS 114 is an intra-hospital/radiology information management system for managing information such as subject information and examination request information used in radiology. The PACS 115 is an image management server aiming for storing and managing images. The PACS 115 includes a data server apparatus for storing images, and a controller 1151 for managing the data. The viewer 116, which is connected with the PACS 115, mainly performs image examination operations, in-depth post-processing, and diagnostic operations on images captured by the X-ray imaging system 101, by using a high definition monitor. The printer 117 prints out X-ray image data and tomosynthesis image data. In this case, the communication circuit 112 of the imaging control apparatus 107 receives examination request information, transmits examination implementation information, and outputs X-ray image data and tomosynthesis image data via the network 113.

The control unit 111 according to the present embodiment is a control apparatus for controlling tomosynthesis imaging using the X-ray detector (image capturing unit) 106. The control unit 111 serves as an imaging apparatus for performing both tomosynthesis imaging control and captured oblique image output control.

The control unit 111 includes a condition setting unit 122 for setting tomosynthesis imaging condition, and an imaging control unit 123 for generating a signal for controlling the sensor array for the X-ray detector (image capturing unit) 106 based on the imaging condition. The control unit 111 further includes a display control unit 124 for controlling the display unit 109 to display a tomographic image including an oblique image based on a plurality of projection images captured by the sensor array controlled by the control unit 111. The control unit 111 further includes an output specification unit 125 for specifying the oblique image, and an information acquisition unit 126 for acquiring information of the position and the orientation of the specified oblique image.

The condition setting unit 122 sets information such as the frame rate, the image size, and the resolution, as an imaging condition, for example, according to an input operation from a user.

The imaging control unit 123 integrally controls the X-ray detector 106 and the X-ray generation apparatus 102. The communication circuit 112 outputs a signal for capturing projection images to the X-ray detector 106 in response to an instruction signal generated by the imaging control unit 123. This output signal includes an instruction for applying a bias voltage to the sensor array of the X-ray detector 106, and an instruction for changing the imaging mode such as fluoroscopic imaging and still-image capturing. For example, the imaging control unit 123 acquires information for specifying pixels from which signals are to be read as an X-ray image, corresponding to the set image size information. The imaging control unit 123 further converts the resolution information into information of the presence or absence of binning drive for adding a plurality of pixels to the output corresponding to 1 pixel, and a range of pixels subjected to binning (e.g., 2 x 2, 3 x 3). The imaging control unit 123 transmits these pieces of information to the X-ray detector (image capturing unit) 106 via the communication circuit 123. Based on the transmitted information, the X-ray detector 106 controls the timing of turning the switch element of each pixel ON and OFF in a drive circuit for driving the sensor array according to information for specifying pixels from which signals are to be read as an X-ray image. Further, the X-ray detector 106 performs control to operate the switch element for adding pixel outputs for binning in the sensor array and a column adding circuit included in a reading circuit for amplifying the output from the sensor array.

The imaging control unit 123 further transmits information of the X-ray generation rate (frame rate) by the X-ray control unit 104 to the X-ray detector 106 and the X-ray control unit 104. The imaging control unit 123 further transmits information of an irradiation condition such as the tube current, the tube voltage, the irradiation time to the X-ray control unit 104. The imaging control unit 123 further transmits information of the irradiation angular range (±θ) of the X-ray generation apparatus 102 and the moving range of the X-ray detector 105 by the moving mechanism control unit 1051 to the moving mechanism control unit 1051. Upon reception of a signal including the information, the X-ray detector 106, the X-ray control unit 104, and the moving mechanism control unit 1051 waits for completion of setting according to the information included in the signal and the start of X-ray imaging triggered by the irradiation switch 103.

Based on a plurality of projection images acquired by X-ray imaging, a tomographic image is generated, for example, through the filtered back projection processing or the iterative approximation reconstruction processing by the image processing unit 111. The tomographic image to be generated may be a plurality of coronal images (or axial images or sagittal images) having different cross sections, or volume data including a plurality of voxels in a predetermined three-dimensional region.

The output specification unit 125 specifies as an output target at least one oblique image generated from the tomographic image. With the specification processing, the user is able to specify a desired oblique image by specifying a predetermined oblique cross section according to the user's input operation while changing the angle with respect to the coronal cross section of the oblique cross section (the detection surface of the X-ray detector 106) in steps of a predetermined angle. Alternatively, for example in a case of re-imaging, when the isocenter position is known and the angle with respect to the detection surface of the target oblique cross section is known, the user specifies an oblique image based on the angle. This allows omitting the trouble of performing an input operation by the user.

In another embodiment, the output specification unit 125 may also specify an oblique image based on an oblique cross section passing through the position or region in the tomographic image. This position indicates one point on the three-dimensional coordinates. When volume data is reconstructed, a point indicates, for example, a specific voxel in the volume data. When a plurality of two-dimensional tomographic images is reconstructed without reconstructing volume data, the point indicates a point on the two-dimensional tomographic images, a point between two-dimensional tomographic images arranged in the three-dimensional space, or a voxel on virtual volume data. On the other hand, the region refers to a plurality of continuous coordinates on the three-dimensional coordinates, and includes a plurality of voxels in a case of volume data. Actually, both a position and a region are included in the concept of "region" in a theoretical meaning. This enables specifying an oblique image for observing a target position or region, allowing the user to easily specify an oblique image useful for diagnosis.

In still another embodiment, the output specification unit 125 may specify a plurality of the oblique images as output targets. For example, specifying a plurality of oblique images based on a plurality of different cross sections as output targets enables observing a target position or a target region in a plurality of the different cross sections. In addition to a plurality of the different cross sections, if a plurality of oblique images having different resolutions and image processing conditions can be specified, a plurality of tomographic images having different image processing conditions can also be specified.

Alternatively, in still another embodiment, the communication circuit (output unit) 112 may output not only an oblique image but also a coronal image together with the oblique image. In this case, the output specification unit 125 specifies at least one oblique image and at least one coronal image as output targets.

When a plurality of cross-sectional images is specified as described above, with the specification of a target point (position), a coronal image or an oblique image passing through the target point may be specified even without directly selecting a cross-sectional image. In still another embodiment, with the specification of a target region, a coronal image or an oblique image passing through the target region may be specified. Alternatively, with the specification of a target point or a target region, a plurality of coronal images and oblique images (or icons indicating cross sections corresponding to these images) may be displayed as output target candidates, and by specifying from among the candidates, an output target oblique image may be specified. In this configuration, since the user only needs to narrow down output target oblique images by performing an input operation, it is possible to improve operation efficiency.

When a plurality of tomographic images is output as described above, for each of at least one oblique image and coronal image specified as an output target, the control unit 111 includes identification information common to a plurality of the oblique images as associated information in a DICOM image. The common identification information is convenient because it allows an output destination apparatus to integrally manage and display a plurality of the DICOM images. The common identification information may be such information as an identifier (ID). In addition to or instead of the ID information, information indicating a common target region or target point (position) through which cross sections corresponding to a plurality of the DICOM image pass is included as the associated information (DICOM tag). The communication circuit (output unit) 112 is assumed to output a plurality of DICOM images including the information. This enables the output destination apparatus to easily observe the target position or target region.

In the above-described embodiments, the output specification unit 125 may specify at least one oblique image, specify at least one coronal image, or specify at least one oblique image and at least one coronal image.

Even in a case where only a single oblique image or a single coronal image is specified, the above-described ID information and the information of the target region or target position may be included in the DICOM image as the associated information.

In many of the above-described embodiments, a coronal image is used as a tomographic image since the body axis of the subject is positioned parallel with the detection surface of an imaging system as illustrated in Fig. 1. However, an axial image and a sagittal image can be captured depending on the layout of the imaging system and the subject. In this way, the embodiments of the present invention also include a case where the output specification unit 125 specifies at least one tomographic image of at least any one of three orthogonal cross sections including the coronal cross section, the axial cross section, and the sagittal cross section.

When the volume data is reconstructed and the target region (three-dimensional region) is specified, the communication circuit (output unit) 112 may output partial volume data of the target region together with the oblique image or coronal image. The partial volume data is generated, for example, by being clipped from the volume data reconstructed by the image processing unit 110. With this processing, the target region can be suitably observed.

When a target region or target position is specified, the image processing unit 110 may generate an image three-dimensionally representing the reconstructed subject, and the communication circuit (output unit) 112 may output the image together with the DICOM image to indicate the position of the target region on the subject. For example, when the volume data has been reconstructed, an image three-dimensionally representing the subject refers to reduced volume data that is the reconstructed volume data reduced, for example, to 1/32 or 1/128. When a tomographic image along a plurality of detection surfaces has been reconstructed, the image refers to pseudo volume data generated from the tomographic image in a pseudo way, of which the data volume is reduced to 1/128.

The image three-dimensionally representing the subject is used together with the information of the target position or target region. For example, the output side changes the display format of the target position or target region from the display format of other regions or positions, for example, by coloring the target position or target region, or displays the target position or target region distinguishably from other regions or positions in the image. In this way, the position of the target region can be more suitably used for diagnosis.

The information acquisition unit 126 acquires the information of the specified position and orientation of a specified oblique image or coronal image. Information indicating the position and the orientation can be represented by three-dimensional coordinates with reference to the isocenter position. For example, in a case of an oblique cross-sectional image that passes through the isocenter and makes an angle Θ1 with the detection surface, the information acquisition unit 126 acquires the angle Θ1 as information of the position and the orientation of the oblique image. Further, in a case of a coronal cross-sectional image separated from the isocenter by a distance z1 in a direction normal to the detection surface and parallel to the detection surface, the information acquisition unit 126 acquires the distance z1 as information of the position and the orientation of the coronal image. In the above described case, the information of the position and the orientation is represented by a vector (θ, z) including, for example, an angle θ made with the detection surface and a distance z from the detection surface.

The oblique image is transmitted from the communication circuit (output unit) 112 to the PACS 115 via the network 113 as a DICOM image including the above-described set imaging condition and the position and the orientation of the specified oblique image, as associated information. An oblique image is an image that is reconstructed and secondarily acquired from projection images directly acquired through imaging. Therefore, information indicating "SECONDARY CAPTURE IMAGE" (secondary capture) is supplied to an Image Type tag (associated information), which indicates a method for acquiring an image having a data format "DIGITAL X-RAY IMAGE INFORMATION OBJECT" defined by the DICOM image.

In addition, the X-ray imaging system 101 may include the imaging table 105 on which the subject is mounted. The imaging table 105 is not required, for example, in a case of tomosynthesis imaging in a standing position. The imaging table 105 may be provided with a storage unit for storing the X-ray detector 106 and a moving mechanism for moving the X-ray detector 106 by moving the position of the storage unit. In tomosynthesis imaging in which the X-ray detector 106 is not moved, the moving mechanism is not required.

The PACS 115 is connected with the X-ray imaging system according to the present embodiment, and receives a DICOM image including an oblique image and tomographic images of the three orthogonal cross sections from the X-ray imaging system 101. In the present embodiment, the control unit 111 of the X-ray imaging system 101 is assumed to include identification information for determining processing by the PACS 115 in the associated information of these DICOM images. As the identification information, the control unit 111 uses, for example, an integer type parameter for defining an image storage period on the PACS 115. In this way, the control unit 111 generates, for example, a DICOM image including an oblique image specified as an output target, and including first identification information as associated information. The control unit 111 further generates a DICOM image including tomographic images of the three orthogonal cross sections, and including second identification information different from the first identification information as associated information. The controller 1151 of the PACS 115, for example, functions as a storage setting unit in which the output specification differentiates the storage period or the storage image format between the DICOM image to be provided with the first identification information, and the DICOM image to be provided with the second identification information different from the first identification information. For example, the identification information of 3 or 1 defines a storage period of 3 years or 1 year, respectively. Alternatively, a storage period may be defined in such a way that the identification information of 1 defines a storage period of 2 years, the identification information of 2 defines a storage period of 5 years, and the identification information of 3 defines a permanent storage period until a specific instruction is given. The identification information may be manually set by the user via an input operation on the operation unit 108, or automatically set by the control unit 111. For example, for an oblique image specified as an output target, 2 is set as the identification information to define a storage period of 5 years, assuming that the image is suitable for diagnosis of the subject. For images of the three orthogonal cross sections, 1 is set as the identification information to define a storage period of 2 years, which is shorter than that of the oblique image. Alternatively, in still another embodiment, when images of the three orthogonal cross sections, for example, a plurality of coronal images (about 10 to 20 adjacent images) is specified as output targets, a longer storage period than that for an oblique image is set on the premise that an oblique image can be generated from the coronal image. Automatically setting the identification information in this way enables reducing user's trouble on management of images.

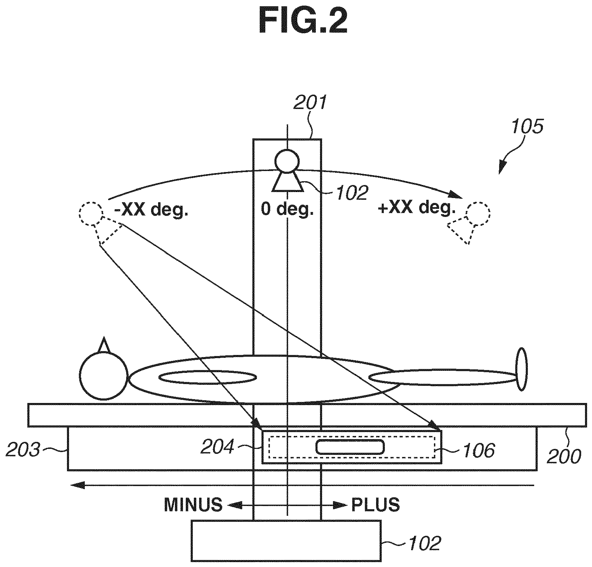

An example of a configuration of the imaging table 105 used for tomosynthesis imaging will be described below with reference to Fig. 2. The imaging table 105 includes a tabletop 200 for holding the subject, a column 201 for holding the X-ray generation apparatus 102, a base 202 of the column 201, a first moving mechanism for moving the X-ray generation apparatus 102, and the moving mechanism control unit 1051 for controlling the moving mechanism. The column 201 can be inclined with respect to the base 202. The X-ray generation apparatus 102 is fixed to the column 201. When projection image data in tomosynthesis imaging is collected, the X-ray generation apparatus 102 moves in the longitudinal direction of the tabletop 200 centering on the position where the tabletop 200 and the column 201 are perpendicular to each other before irradiation is started.

The imaging table 105 may further include a supporting portion 204 for supporting the X-ray detector 106, a guide portion 203 of the supporting portion 204, and a second moving mechanism for moving the X-ray detector 106. In this case, the supporting portion 204 moves along the guide portion 203 by a preset distance in the longitudinal direction of the tabletop 200 and in the opposite direction of the X-ray generation apparatus 102. In this way, when irradiation is started, the X-ray imaging system 101 collects projection image data and acquires position information as a basis of the reconstruction processing while the X-ray generation apparatus 102 and the X-ray detector 106 are moving in opposite directions to each other.

Although the X-ray detector 106 draws a linear trajectory and the X-ray generation apparatus 102 draws a circular arc trajectory, the X-ray generation apparatus 102 may also draw a linear trajectory. In this case, the first moving mechanism may move the X-ray generation apparatus 102 in a direction perpendicular to the major-axis direction of the column 201 while suitably changing the position of the X-ray generation apparatus 102 with respect to the major-axis direction of the column 201.

The first and the second moving mechanisms for moving the column 201 and the supporting portion 204 of the X-ray detector 106 are controlled by the moving mechanism control unit 1051. The moving mechanism control unit 1051 moves the X-ray generation apparatus 102 and the X-ray detector 106 so that they are disposed each at a predetermined position, at a predetermined timing, and at a predetermined speed according to input control parameters.

In addition, the angle of the tabletop 200 of the imaging table 105 in the gravity direction can be changed. In a case of imaging in a standing position, the tabletop 200 is inclined to a direction along the gravity direction, and functions as a supporting portion for supporting the subject.

In addition, the imaging table 105 used for tomosynthesis imaging can take other embodiments, for example, so as to move the X-ray generation apparatus 102.

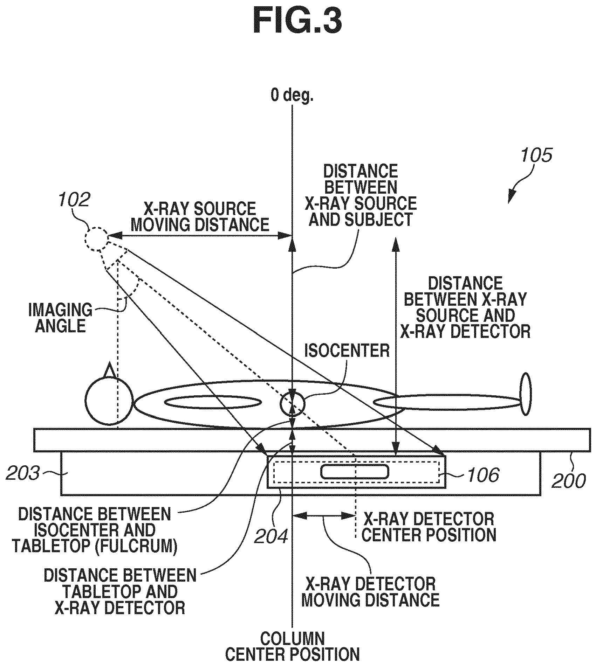

Fig. 3 illustrates information of the geometric arrangement (geometry) in the tomosynthesis imaging. The information is acquired, for example, when projection image data is collected, and is transmitted from the moving mechanism control unit 1051 to the imaging control apparatus 107.

The isocenter refers to a center position used when tomosynthesis imaging is performed. Normally, depending on the reconstruction method, the coronal image passing through the isocenter provides the highest image quality. Therefore, desirably, the isocenter is determined before starting imaging so that the coronal image passes through the inside of the target region of the subject. The X-ray generation apparatus 102 and the X-ray detector 106 respectively move so that the line connecting the X-ray focal point of the X-ray generation apparatus 102 and the center (detector center position) of the detection surface of the X-ray detector 106 constantly passes through the isocenter. The isocenter position is set, for example, in terms of the distance from the upper surface of the tabletop 200 of the imaging table 105 (the distance between the fulcrum or the isocenter and the tabletop 200).

The imaging angle or the projection angle is an angle made between a straight line connecting the X-ray generation apparatus 102 and the isocenter and the normal line of the detection surface of the X-ray detector 106. The imaging angle is determined before imaging since it is a parameter affecting image quality.

Other parameters affecting the geometry of tomosynthesis imaging include an imaging region or a reconstruction region. The geometry in a direction parallel to the detection surface is determined by the collimator light irradiation region when the X-ray generation apparatus 102 is at the column center position. The geometry in a direction perpendicular to the detection surface may be determined by the height from the isocenter or the height from the tabletop 200. The reconstruction region may be determined by displaying on the display unit 109 a schematic diagram of the subject laid on the tabletop 200, and specifying the three-dimensional region of the subject. Although, in Fig. 3, the front surface of the X-ray detector 106 is irradiated with X-rays, the configuration is not limited thereto. The imaging region may be limited according to an imaging target region.

According to the isocenter, the imaging angle (projection angle), and the reconstruction region determined in this way, a range of the X-ray source moving distance and a range of the X-ray detector moving distance are determined. Although the distance between the tabletop 200 and the X-ray detector 106 is predetermined, it may be variable. These pieces of information are determined, for example, by the imaging control apparatus 107 or input to the imaging control apparatus 107 via the operation unit 108, and then stored.

The number of projection images to be captured is predetermined before imaging. This number determines the amount of movements of the X-ray generation apparatus 102 and the X-ray detector 106 for each image capturing. Accordingly, the irradiation interval of the X-ray generation apparatus 102 will be determined.

The imaging angle, the X-ray source moving distance, and the X-ray detector moving distance change in each projection image capturing. When the X-ray generation apparatus 102 draws a curved trajectory, the distance between the X-ray source and the subject and the distance between the X-ray source and the X-ray detector 106 also change. The moving mechanism control unit 1051 records these parameters at the timing of each projection imaging, i.e., each time X-ray irradiation is made, and transmits the parameters to the imaging control apparatus 107 as geometric information corresponding to each projection image.

Although, in the present embodiment illustrated in Fig. 3, the column 201 inclines at the contact portion with the base 202 as a method for moving the X-ray generation apparatus 102, a method for horizontally moving the column 201 may be used.

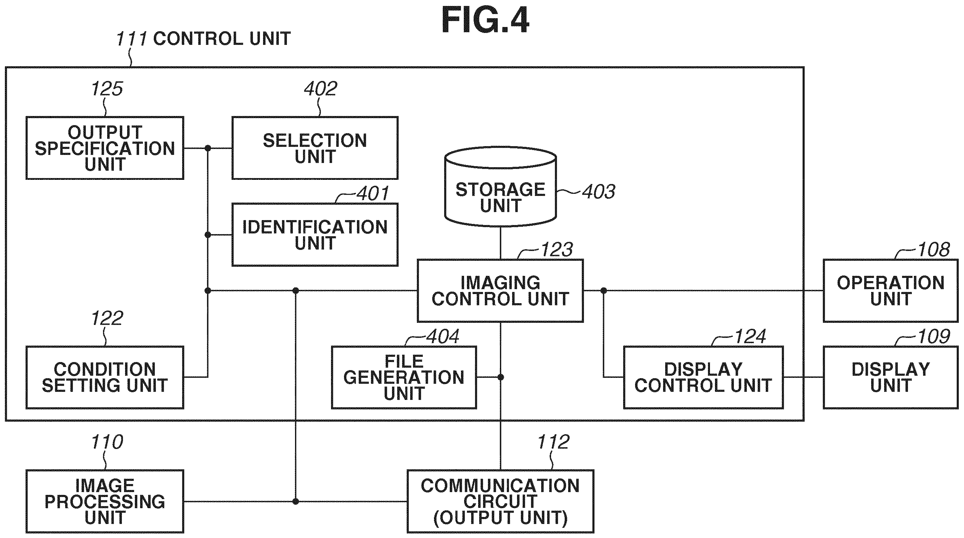

Fig. 4 illustrates an example configuration of the control unit 111 in detail according to the present embodiment. In addition to the condition setting unit 122, the imaging control unit 123, the display control unit 124, the output specification unit 125, and the information acquisition unit 126 described above, the control unit 111 includes an identification unit 401 and a selection unit 402 for identifying a position or region in a tomographic image, a storage unit 403 for storing examination information, and a file generation unit 404 for generating a DICOM file.

The identification unit 401 stores the three-dimensional coordinates of a target point in a three-dimensional reconstruction memory and the upper-limit number of specific target points based on the coordinates on a coronal image specified based on an input from the operation unit 108 and the height of the coronal image. The selection unit 402 selects one through three points for specifying an oblique cross section out of the target points stored in the identification unit 401.

The storage unit 403 stores, updates, deletes, and searches for imaging technique information. The imaging technique information includes various information such as information for specifying an imaging technique (such as an imaging portion and an imaging direction), the default imaging condition, default image processing parameters, default reconstruction parameters, a storage transmission setting, a print output setting, and all other setting items which can be set for each imaging technique ranging from imaging implementation to post-processing and image output setting. The storage unit 403 includes a database, and registers, updates, deletes, and searches for examination information.

The imaging control unit 123 transmits and receives such data as imaging permission/prohibition, an imaging implementation condition, and position information to and from the X-ray generation apparatus 102 and the X-ray detector 106 via the communication circuit 112. The imaging control unit 123 further performs an overall reconstruction processing implementation flow including a one-time X-ray imaging flow such as X-ray image data storage. The imaging control unit 123 further performs an overall examination implementation flow including update and registration control (on patient information, scheduled examination information, and imaging technique information), screen transition control, and processing for storing tomosynthesis image data and adding tomosynthesis images. The imaging control unit 123 further performs control on input information reception from the operation unit 108 and display control of the screen shift on the display unit 109. The imaging control unit 123 further determines the image output permission/prohibition on an image included in the received examination information, and instructs the communication circuit 112 to output the image.

The output specification unit 125 specifies as an output target an oblique image or a coronal image specified via an input operation on the operation unit 108.

The DICOM image generation unit 404 generates a DICOM image including as image data an oblique image specified by the output specification unit 125. The DICOM tag including the associated information of the DICOM image is provided with information of the position and the orientation of the oblique image acquired by the information acquisition unit 126, the imaging condition set by the condition setting unit 122, and information indicating that the image data is "SECONDARY CAPTURE IMAGE" (secondary capture).

The section passing through the isocenter is a cross section perpendicularly intersecting with the irradiation direction in projection image capturing, and provides a reconstructed tomographic image with high image quality. However, depending on the irradiation angle in projection image capturing, X-ray irradiation from a direction perpendicular to the oblique cross section may not be performed. In this case, it is considered to be difficult to improve image quality of an oblique image of the oblique cross section. Therefore, in one embodiment, the imaging control unit 123 determines whether at least any one of image quality of the above-described oblique image and the position of the above-described cross section satisfies a predetermined criterion. When the imaging control unit 123 determines that image quality of the oblique cross section does not satisfy the image quality criterion, the display control unit 124 controls the display unit 109 to display the oblique image together with a message notifying of the situation. This enables notifying an operator (i.e., person who has taken the oblique image) of insufficient image quality of the oblique image to draw the operator's attention, reducing the possibility that the image having insufficient image quality is unsuitably used. In still another embodiment, the display control unit 124 performs control not to display the oblique image that does not satisfy the image quality criterion, to reduce the possibility that the oblique image having insufficient image quality is unsuitably used. The determination of image quality of the oblique image is based on the relation between the cross section of the oblique image and the size range of each projection image in the irradiation direction, image quality of the oblique image, and other various criteria. As described above-, according to the result of the determination by the imaging control unit 123, the display control unit 124 restricts the oblique image display or displays a warning about the oblique image, thereby reducing the possibility that the oblique image is unsuitably used.

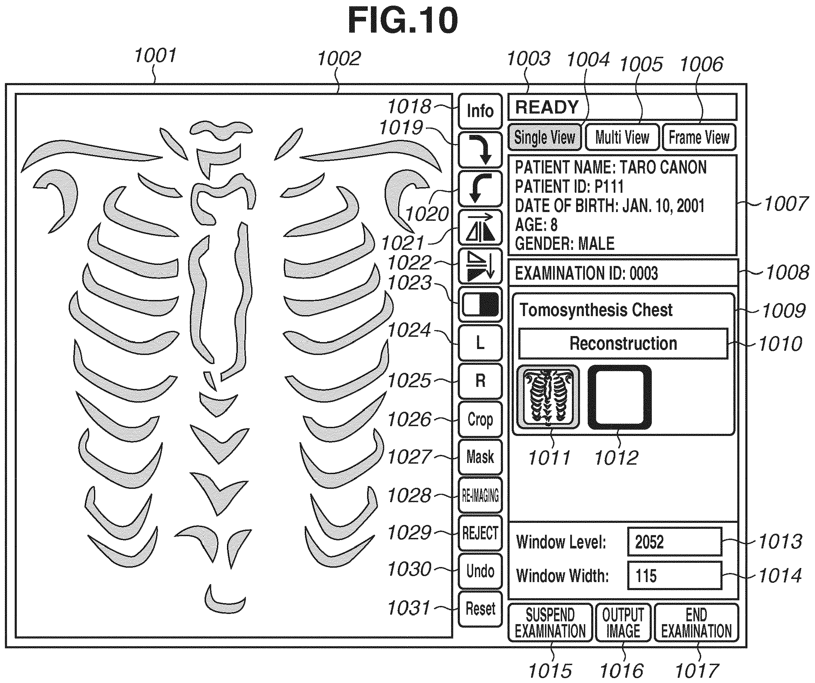

Alternatively, when the imaging control unit 123 determines that image quality of the oblique image does not satisfy the image quality criterion, the re-imaging may be performed through the geometry which enables reconstructing the oblique image while satisfying the image quality criterion. In this case, the imaging control unit 123 generates an imaging condition based on the irradiation angular range of the X-ray generating apparatus 102 suitable for capturing the oblique image, or on a cross section where the isocenter position includes the oblique image. For example, when the angle made between a cross section (including the specified position and the isocenter position) and a direction perpendicular to the detection surface of the X-ray detector 106 is Θ1, the X-ray generation apparatus 102 irradiates the subject with X-rays while the X-ray generation apparatus 102 is being moved within an irradiation angular range exceeding the angle Θ1. Therefore, the X-ray irradiation angular range is set to ±θ1 or more. Alternatively, when two positions are specified, the imaging control unit 123 sets the isocenter position or the moving direction of the X-ray generation apparatus 102 so that the line segment connecting the two positions and the isocenter line are in parallel. When the moving path of the X-ray generation apparatus 102 is a predetermined circular arc trajectory, and the isocenter parallel to the line segment connecting the specified positions cannot be set, the imaging control unit 123 moves the direction of the body axis of the subject by -θ2 with respect to the X-ray imaging system 101, where θ2 is an angle made between the line segment and the isocenter line. This achieves imaging for capturing an oblique image including the specified position. In this case, the display control unit 124 controls the display unit 109 to display the imaging condition, the irradiation condition, or the change amount of the subject orientation. Through the processing, supporting changes of the imaging condition can be performed. Alternatively, after the imaging control unit 123 generates the imaging condition, the display control unit 124 controls the display unit 109 to display an icon indicating the imaging information corresponding to the changed imaging condition. The imaging information is displayed as reservation information for tomosynthesis imaging. For example, in an imaging screen 1001 illustrated in Fig. 10, a new imaging technique display area 1009 is displayed directly under an imaging technique display area 1009 which has been already displayed.

In response to an input operation of pressing the icon, the condition setting unit 122 sets the imaging condition corresponding to the imaging information as a condition to be used for imaging. The imaging control unit 123 outputs the imaging condition of tomosynthesis imaging of the above-described re-imaging including changed information of the isocenter position and the X-ray irradiation angular range, to the X-ray control unit 104 and the moving mechanism control unit 1051. The output is performed via the communication circuit 112. Accordingly, each of the X-ray control unit 104 and the moving mechanism control unit 1051 sets the changed imaging condition to easily achieve re-imaging.

In still another embodiment, when the imaging control unit 123 determines that the oblique cross-sectional image does not satisfy the predetermined image quality criterion, the image processing unit 110 generates an X-ray image corresponding to projection images acquired through scout imaging for tomosynthesis imaging as re-imaging. The X-ray image is an image reproducing in a pseudo manner each projection image (scout image) acquired through scout imaging, and is generated based on a plurality of projection images. Scout imaging refers to fluoroscopic imaging (moving image capturing) for positioning performed before projection image capturing. Scout imaging is performed so that the irradiation direction of the X-ray generation apparatus 102 coincides with a normal line of the detection surface of the X-ray detector 106. Therefore, a fluoroscopic image in suitable scout imaging can be generated in a pseudo manner by sequentially projecting captured tomographic images. For example, suppose a case where the isocenter position needs to be shifted by L1 in a direction parallel to the detection surface. In this case, for example, the subject position needs to be shifted by L1. Then, after shifting the subject position by L1, a pseudo image corresponding to projection images acquired through scout imaging is generated from tomographic images. When the imaging region for scout imaging is a square having one side length of T, an image in which the tomographic images are sequentially projected in a direction perpendicular to the detection surface is acquired. A pseudo image is generated by clipping from the acquired image an image of the square region having one side length of T, centering on a position shifted from the center of the acquired image by L1. The processing is performed by the image processing unit 110. Alternatively, when only shifting the subject position in a direction parallel to the detection surface in this way, a pseudo image may also be generated by clipping an imaging region corresponding to scout imaging from projection images acquired by X-ray irradiation from a direction perpendicular to the detection surface.

For example, suppose a case where the tabletop 200 of the table 105 can be inclined with respect to the vertical direction. Suppose a case of tomosynthesis imaging where the X-ray generation apparatus 102 has been moved in a range of ±θ1, the inclination of the target oblique image with respect to the detection surface is -θ2 (θ2 > θ1), and the movable range of the X-ray generation apparatus 102 is ±(θ1 <) θ3 (< θ2). In this case, when the tabletop 200 is inclined at least by Δθ = θ2 - θ3 and the moving range of the X-ray generation apparatus 102 is set to ±θ3, the X-ray generation apparatus 102 will move in a range from -θ2 to 2 x θ3 - θ2 with respect to the tabletop 200. Thus, image quality of the oblique images will be guaranteed by generating tomographic images from projection images acquired in tomosynthesis imaging by using the above-described imaging system, and generating an oblique image having an angle -θ2 with respect to the detection surface. Since the imaging system emits X-rays from a direction perpendicular to the oblique image, projection images parallel to the oblique image are used for reconstruction. Inclining the tabletop 200 by an angle larger than Δθ improves image quality of the oblique image to be acquired.

A scout image acquired in a state where the tabletop 200 is inclined as described above becomes parallel to projection images acquired in X-ray irradiation from a direction Δθ with respect to that in a state where the tabletop 200 is not inclined. Therefore, the imaging system identifies each projection image, acquired through X-ray irradiation from the direction Δθ in the first tomosynthesis imaging, as original data of the scout image to be generated. A pseudo scout image is generated by clipping a region corresponding to scout imaging from projection images of the original data. When X-rays are not strictly emitted from the direction Δθ, the imaging system identifies projection images acquired through X-ray irradiation from a direction closest to Δθ as original data of the scout image. In this way, when the display control unit 124 controls the display unit 109 to display a pseudo image, for example, an engineer is able to suitably perform fluoroscopic imaging for positioning referring to the image.

As described above, the display control unit 124 performs display control for re-imaging according to the determination that the above-described predetermined image quality criterion is not satisfied to enable supporting re-imaging for acquiring a desired oblique image.

The storage unit 403 stores threshold values of the difference between images for calculating the difference between the oblique image and the coronal image passing through a rotation center line. The imaging control unit 123 calculates the difference between the oblique image acquired from the coordinates of two to three target points stored in the identification unit 401 and the coronal image passing through the rotation center line. The imaging control unit 123 compares the calculated difference with the threshold values stored in the storage unit 403. According to the result of the comparison, the display control unit 124 displays a warning indicating that image quality of the oblique image is not sufficient or restricts the oblique image display.

The difference between images may be the oblique angle, i.e., an angle made by the coronal image and the oblique image, or the distance between the point where the normal line passing through the center of the coronal image intersects with the oblique image and the rotation center line. Further, the difference may be a combination of the angle and the distance.

The imaging control unit 123 calculates the maximum projection angle, i.e., the maximum angle of a plurality of captured projection images captured based on position information notified from the X-ray control unit 104 and the X-ray detector 106. Then, the imaging control unit 123 sets the calculated maximum projection angle to a first threshold value or a second threshold value or both of the oblique angles stored in the storage unit 403.

Respective threshold values may be common to all imaging techniques, may be stored for each imaging technique, or may be changed by the operator.

One threshold value may be stored, and the display control unit 124 may display a warning or restrict the oblique image display. Two threshold values may be stored, and the display control unit 124 may display a warning when the oblique angle is between the first and the second threshold values, or to restrict the oblique image display when the oblique angle exceeds the second threshold value. Further, the display control unit 124 may recommend the operator to perform re-imaging through tomosynthesis imaging after changing the imaging condition so that the oblique image passes through the rotation center line. When the oblique image is close to a sagittal image (vertically cut cross-sectional image), the display control unit 124 may recommend the operator to perform re-imaging through tomosynthesis imaging after changing the subject position to the lateral or oblique position. When the oblique image is close to an axial image (cross-sectional image in the body slicing direction), the display control unit 124 may recommend the operator to perform re-imaging through other modality imaging such as Computer Tomography (CT) and Magnetic Resonance Imaging (MRI).

When re-imaging through tomosynthesis imaging is recommended, the imaging condition may be such that the rotation center line intersects with the oblique image by changing only the height direction, or that the rotation center line passes through the centroid of two to three target points (the midpoint in the case of two points).

The storage unit 403 stores the first threshold value of the oblique angle for displaying a warning according to the value of an input oblique angle, and the second threshold value of the oblique angle for restricting the oblique image generation or display when the input oblique angle is excessively large. When either one of the two threshold values is used, only one may be stored. Respective threshold values stored in the storage unit 403 may be common to all imaging techniques, may be stored for each imaging technique, or may be changed by the operator.

The imaging control unit 123 calculates the maximum projection angle, i.e., the maximum angle of a plurality of captured projection images captured based on position information notified from the X-ray control unit 104 and the X-ray detector 106. Then, the imaging control unit 123 sets the calculated maximum projection angle to the first or the second threshold value or both of the oblique angles stored in the storage unit 403.

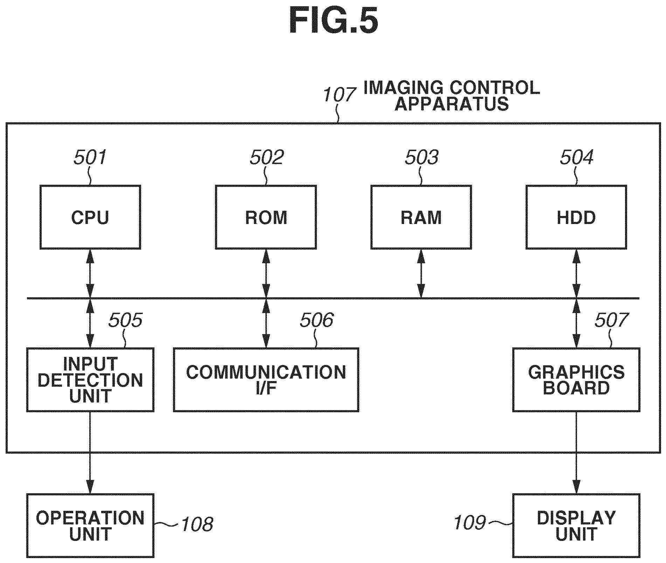

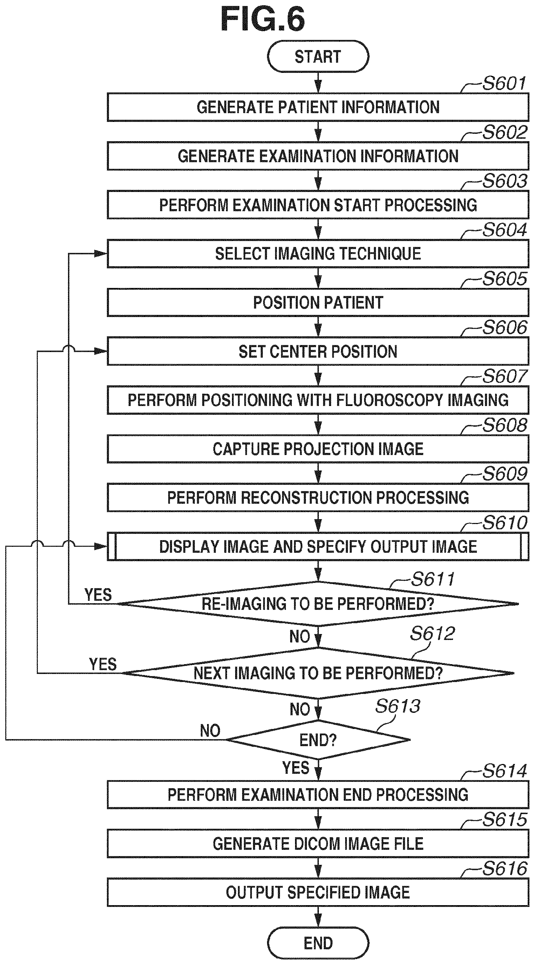

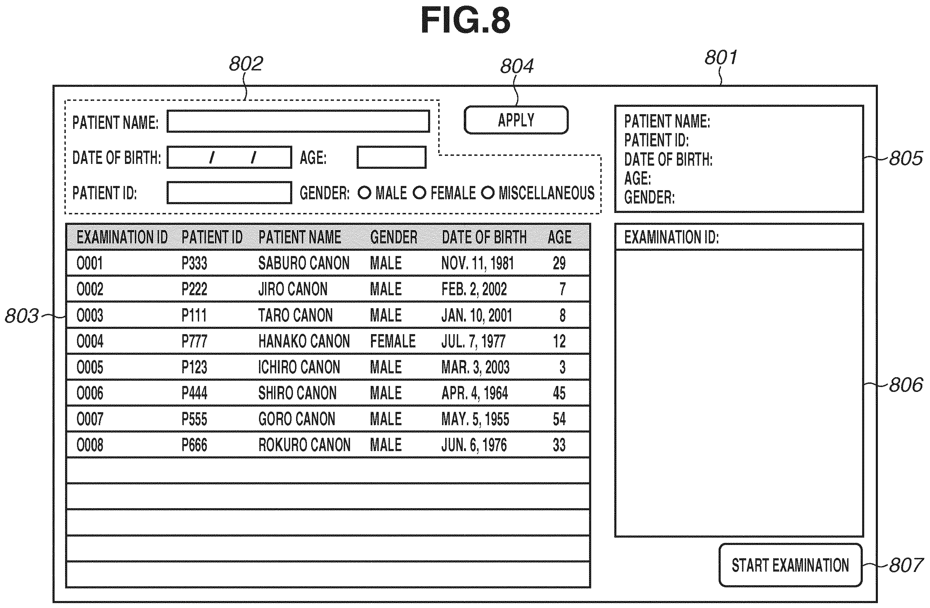

Fig. 5 illustrates a hardware configuration of the control unit 111 relating to the X-ray imaging system 101 according to the present invention. The control unit 111 includes a central processing unit (CPU) 501, a read only memory (ROM) 502, a random access memory (RAM) 503, a hard disk drive (HDD) 504, an input detection unit 505, a communication I/F 506, and a graphics board 507. These units are connected with each other via a bus such as a data bus. The hardware of the control unit 111 may be a commercial electronic computer. Each function of the control unit 111 is implemented when a program including instructions for implementing the processing illustrated in Figs. 6, 7, and 21 according to the present embodiment, and data for implementing the graphical user interfaces (GUIs) illustrated in Figs. 8 to 20, and 23 are installed in the electronic computer. The CPU 501 is a calculation processing circuit for integrally performing control by the control unit 111. The CPU 501 implements control by executing an instruction program stored in the ROM 502 or a program stored in the HDD 504 and loaded into the RAM 503. The CPU 501 further performs processing for setting the imaging condition of tomosynthesis imaging. The CPU 501 instructs the graphics board 507 to perform display control on the display unit 109, i.e., the graphics board 507 controls the display unit 109 to display tomographic images including an oblique image based on a plurality of projection images acquired by a sensor. The CPU 501 further controls an input operation performed on the operation unit 108 via the input detection unit 505, and performs processing for specifying an oblique image and processing for acquiring information of the position and the orientation of the specified oblique image. The RAM 503 is also used to ensure a working storage area when the CPU 501 performs control by an instruction program. The HDD 504 is an auxiliary storage device for storing various data such as X-ray image data. The communication I/F 506 is a communication interface configuring the communication circuit 112. The communication I/F 506 exchange data between the control unit 111 and the X-ray control unit 104, the X-ray detector 106, and the network 113. The communication I/F 506 transmits a signal for controlling the sensor array to the X-ray detector 106 based on a set imaging condition, and functions as a receiver for receiving and acquiring a plurality of projection images from a radiation detector used for tomosynthesis imaging. The communication I/F 506 outputs a DICOM secondary capture image including the specified oblique image as an image acquired in the above-described tomosynthesis imaging, and including as associated information the information of the above-described imaging condition and information for identifying the position and the orientation of the above-described oblique image. The communication I/F for communicating with the X-ray detector (image capturing unit) 106 and the communication I/F for outputting a DICOM image may be identical hardware or respectively different network interface cards.

The graphics board 507 serves as at least a part of the image processing unit 110 and a display driver for performing the function of the display control unit 124. The graphics board 507 includes a Graphic Processing Unit (GPU), and performs image processing by the GPU, the reconstruction processing, and processing for generating and displaying an oblique cross section according to an input operation. For example, the graphics board 507 functions as a graphic processor for displaying an oblique image of a cross section including a specified position and the isocenter for tomosynthesis imaging on a display. A part of image processing may be performed by the CPU 501. In this case, the CPU 501 and the graphics board 507 correspond to the image processing unit 110.

Fig. 6 illustrates an example of a processing flow from the start to the end of tomosynthesis imaging examination according to the present embodiment.

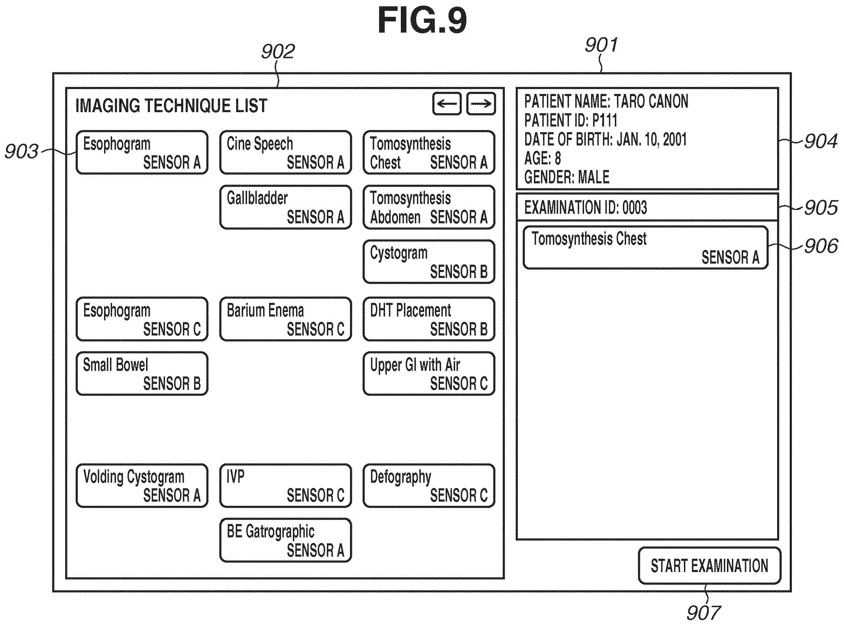

In step S601, the imaging control unit 123 generates patient (subject) examination information. Before starting an examination, patient information is generated. The patient information includes all of information for specifying a patient, such as a patient name, patient ID, age, date of birth, gender, height, weight, and pregnancy state. The display control unit 124 displays a patient information input screen 801 (see Fig. 8) on the display unit 109. The imaging control unit 123 identifies examination target patient information according to an input operation performed on the operation unit 108. Accordingly, the imaging control unit 123 generates newly scheduled examination information. The scheduled examination information includes the above-described patient information, the examination information for specifying an examination, such as an examination ID, examination date, and imaging technique information including all of the information for specifying an imaging technique, such as an imaging portion. The imaging control unit 123 inputs the patient information included in a patient information determination notification to the scheduled examination information. Then, the imaging control unit 123 requests all of the imaging technique information registered in the storage unit 403, and acquires all of the registered imaging technique information. Upon reception of the imaging technique information, the imaging control unit 123 instructs the display control unit 124 to display an imaging technique selection screen 901 (see Fig. 9). The display control unit 124 displays all of the received imaging technique information on the imaging technique selection screen 901.

In step S602, the imaging control unit 123 generates the examination information. The generated examination information also includes scheduled imaging techniques. Upon reception of an examination start instruction from the operation unit 108, the imaging control unit 123 inputs the examination information and the scheduled imaging technique to the scheduled examination information generated when the patient information is determined.

Although, in the present embodiment, the patient information, the examination information, and the scheduled imaging techniques are manually generated, the patient information, the examination information, and the scheduled imaging techniques may be generated at one time by selecting work list information acquired from the HIS/RIS 114 in still another embodiment. In this case, step S601 is skipped. In response to an input operation for issuing an examination start instruction performed on the operation unit 108, the imaging control unit 123 inputs the patient information, the examination information, and the scheduled imaging techniques included in the selected work list information to the scheduled examination information generated at the time of patient information determination.

In step S603, the imaging control unit 123 performs examination start processing. When the generation of the examination implementation information is completed in step S602, the imaging control unit 123 registers the scheduled examination information as new examination information. Then, the storage unit 403 updates the examination status of the newly registered examination information to "Examination in Progress". The examination status includes "Examination Not Started", "Examination in Progress", "Examination Suspended", and "Examination Ended". The display control unit 124 displays the imaging screen 1001 (see Fig. 10) relating to the scheduled examination information on the display unit 109. The imaging screen 1001 displays the patient information, the examination information, and the imaging technique information included in the received examination information.

In step S604, the imaging control unit 123 selects an imaging technique to be used for next imaging out of the scheduled imaging techniques included in the started examination information. Accordingly, the condition setting unit 126 sets an imaging condition of the tomosynthesis imaging. An imaging technique is selected when the operator presses the imaging technique display area 1009 displayed in the imaging screen 1001. Upon reception of an irradiation permission request notification in response to an input operation of pressing the imaging technique button performed on the operation unit 108, the imaging control unit 123 instructs the communication circuit 112 to output an irradiation permission request notification (a signal for controlling the sensor) to the X-ray detector 106 based on the imaging condition. Upon reception of the irradiation permission request notification, the communication circuit 112 transmits an irradiation permission request notification to the X-ray control unit 104 and the X-ray detector 106. The display control unit 124 changes the display of a sensor status display area 903 in the imaging screen 1001 displayed on the display unit 109. The irradiation permission request notification includes the selected imaging technique information.

Upon reception of the irradiation permission request notification, the X-ray control unit 104 notifies the X-ray generation apparatus 102 of the default imaging condition, and the default position information included in the imaging technique information included in the irradiation permission request notification. Then, when the X-ray generation apparatus 102 completes condition setting and movement to the default position, the X-ray control unit 104 transmits an irradiation permission notification to the communication circuit 112. The irradiation permission notification includes the imaging technique information for which irradiation is permitted.

Upon reception of the irradiation permission request notification, the X-ray detector 106 moves to the default position based on the default position information included in the imaging technique information included in the irradiation permission request notification. Upon completion of X-ray detection preparation, the X-ray detector 106 transmits an irradiation permission notification to the communication circuit 112. Upon reception of the irradiation permission notification from both the X-ray control unit 104 and the X-ray detector 106, the communication circuit 112 transmits an irradiation permission notification to the imaging control unit 123.

Upon reception of the irradiation permission notification, the imaging control unit 123 transmits an irradiation permission notification to the imaging control unit 123. Upon reception of the irradiation permission notification, the imaging control unit 123 transmits an irradiation permission notification to the display control unit 124. Upon reception of the irradiation permission notification, the display control unit 124 changes the display in the sensor status display area 903 in the imaging screen 1001 displayed on the display unit 109. The display unit 109 further displays an imaging schedule thumbnail 912 in the imaging technique display area 1009 in the imaging screen 1001. In this way, when the display in the sensor status display area 903 and the imaging technique display area 1009 is changed, the operator is able to easily recognize the irradiation ready condition and an imaging technique subjected to image addition in the next irradiation. Although a processing flow for manually selecting an imaging technique has been described above, it is also possible to automatically select an imaging technique at a timing when the next imaging becomes ready, for example, when an examination is started or when irradiation is ended in the present embodiment. In this case, when the next imaging becomes ready, the imaging control unit 123 acquires imaging technique information with the status "Not Captured" out of the scheduled imaging technique information included in the scheduled examination information. The status of the imaging technique information includes "Imaging in Progress" and "Imaging Completed" in addition to "Not Captured". The imaging control unit 123 selects an imaging technique with the first registration order out of imaging technique information with the status "Not Captured", and transmits an irradiation permission request. However, a method for selecting an imaging technique is not limited thereto. This enables saving operator's trouble for manually selecting the next imaging technique each time the operator completes imaging, reducing the workflow.

Before or after steps S601 to S604, the operator (or the person in charge of examination implementation) positions the subject. In step S606, the imaging control unit 123 sets the center position for reconstruction. The operator (or the person in charge of examination implementation) measures the center position (hereinafter referred to as an isocenter position) based on the target region of the subject, and inputs the isocenter position from the operation unit 108. Upon input of the isocenter position from the operation unit 108, the imaging control unit 123 inputs isocenter position information to the position information included in the currently selected imaging technique information.

In step S607, the imaging control unit 123 performs positioning of the subject by using fluoroscopic imaging. The imaging control unit 123 controls the reception and display of the image acquired by the fluoroscopic imaging. Particularly in tomosynthesis imaging, since artifact largely affects depending on the X-ray irradiation direction relative to the subject, the imaging control unit 123 performs fluoroscopic imaging to confirm the positioning of the patient to check whether the position of the subject is correct.

When the operator presses the X-ray irradiation switch 103, the X-ray irradiation switch 103 transmits an irradiation start request to the X-ray control unit 104. Upon reception of the irradiation start request, the X-ray control unit 104 transmits an irradiation start instruction to the X-ray generation apparatus 102. Upon reception of the irradiation start instruction, the X-ray generation apparatus 102 starts X-ray irradiation. Then, the X-ray generation apparatus 102 transmits an irradiation start notification to the X-ray control unit 104. Upon reception of the irradiation start notification, the X-ray control unit 104 transmits an irradiation start notification to the imaging control unit 123 via the communication circuit 112. Upon reception of the irradiation start notification, the imaging control unit 123 updates the status of the imaging technique with which irradiation was started to "Imaging in Progress" out of the imaging technique information included in the scheduled examination information. Then, the imaging control unit 123 transmits an irradiation start notification to the display control unit 124. Upon reception of the irradiation start notification, the display control unit 124 changes the display in the sensor status display area 903 in the imaging screen 1001.

The X-ray detector 106 detects the emitted X-rays, and converts them into X-ray image data. The X-ray detector 106 further acquires the position information in synchronization with the X-ray detection. The X-ray detector 106 transmits the X-ray image data and the position information to the imaging control unit 123 via the communication circuit 112. Upon reception of the X-ray image data and the position information, the imaging control unit 123 inputs the position information to the currently selected imaging technique. The imaging control unit 123 further controls the display control unit 124 to cause a live display of the X-ray image data to be displayed in the image display area 902 in the imaging screen 1001 on the display unit 109.

When the operator releases the X-ray irradiation switch 103, the X-ray irradiation switch 103 transmits an irradiation stop request to the X-ray control unit 104. Upon reception of the irradiation stop request, the X-ray control unit 104 transmits an irradiation stop instruction to the X-ray generation apparatus 102. Upon reception of the irradiation stop instruction, the X-ray generation apparatus 102 stops X-ray irradiation. Then, the X-ray generation apparatus 102 transmits an irradiation end notification and an imaging implementation condition notification to the X-ray control unit 104. The imaging implementation condition notification includes the imaging implementation condition and the position information. Upon reception of the irradiation end notification and the imaging implementation condition notification, the X-ray control unit 104 transmits an irradiation end notification and the imaging implementation condition notification to the imaging control unit 123 via the communication circuit 112. Upon reception of the irradiation end notification and the imaging implementation condition notification, the imaging control unit 123 updates the status of the imaging technique with which irradiation was ended out of the imaging technique information included in the scheduled examination information to "Imaging Completed". The imaging control unit 123 inputs the irradiation implementation condition to the imaging technique information with which irradiation was ended out of the imaging technique information included in the scheduled examination information. The imaging control unit 123 causes the display control unit 124 to change the display in the sensor status display area 903 and to update the corresponding annotation display in the image display area 902 in the imaging screen 1001 displayed on the display unit 109.

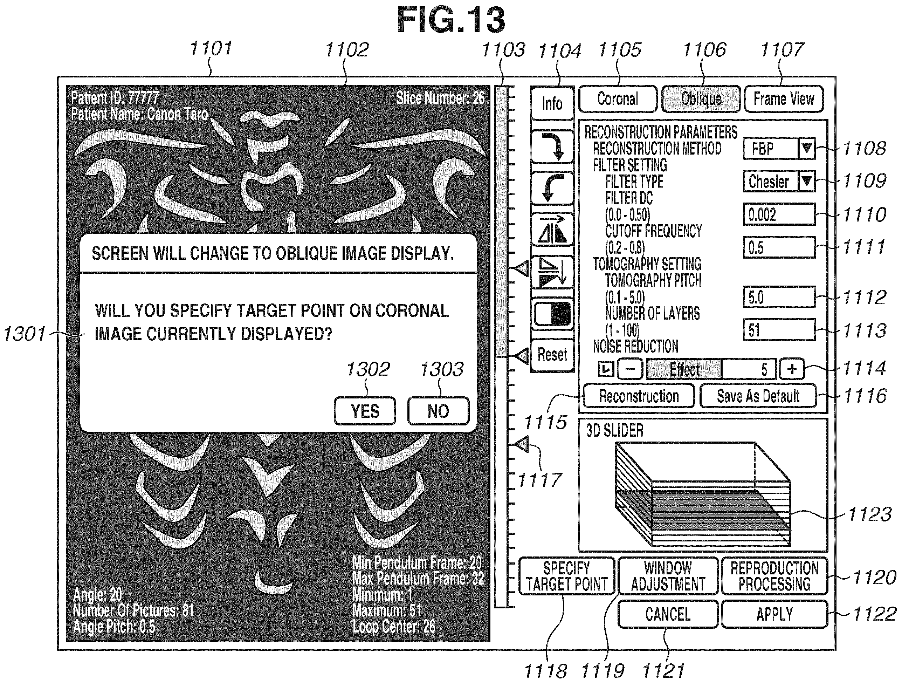

Although, in the above-described case, the X-ray generation apparatus 102 transmits an irradiation end notification and the imaging implementation condition notification at the same time, the present embodiment is not limited thereto. The imaging implementation condition may be transmitted in real time during X-ray irradiation or at a timing different from the timing of the irradiation end notification after the end of X-ray irradiation. Further, the imaging implementation condition and the position information may be transmitted at different timings. In step S608, the imaging control unit 123 captures projection images. The processing flow for capturing projection images is similar to that in the case of fluoroscopic imaging in step S607. However, in projection image capturing, upon reception of the X-ray image data and position information, the imaging control unit 123 inputs the position information to the currently selected imaging technique, and stores the X-ray image data. Upon reception of the irradiation end notification about the projection images, the imaging control unit 123 determines the imaging interruption status based on the position information. The imaging control unit 123 determines the reconstruction processing permission/prohibition or the oblique cross section display permission/prohibition according to the result of the determination of the imaging interruption status (see Fig. 13). This enables preventing useless reconstruction implementation and invalid tomosynthesis image display when the projection image capturing is interrupted. Similarly, referring to the oblique cross section with lacked information enables avoiding the risk of misdiagnosis.

In step S609, the imaging control unit 123 performs the reconstruction processing. Upon reception of the X-ray image data and position information of projection images, the imaging control unit 123 transmits a reconstruction start notification to the display control unit 124 and, at the same time, transmits a reconstruction request notification to the image processing unit 110. The reconstruction request notification includes the imaging technique information, the X-ray image data, and the position information.

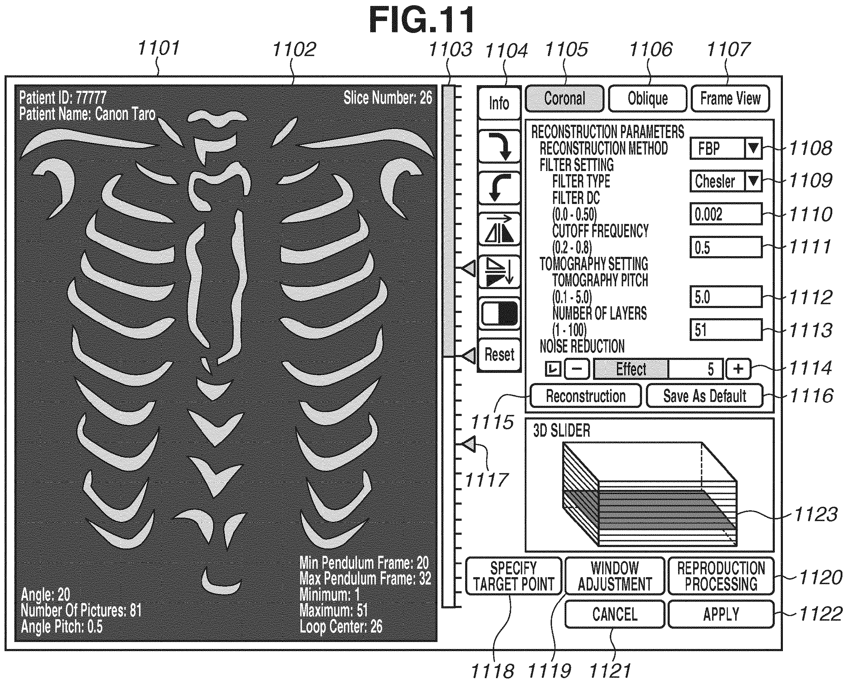

In step S610, the display control unit 124 performs processing for displaying the reconstructed image. Upon reception of the reconstruction start notification, the display control unit 124 displays a reconstruction screen 1101 (see Fig. 11) on the display unit 109, and displays a progress bar in an image display area 1002 in the reconstruction screen 1101. Meanwhile, upon reception of the reconstruction request notification, the image processing unit 110 performs the reconstruction processing by using the default reconstruction parameters, the position information, and the X-ray image data of the imaging technique information. Upon completion of the reconstruction processing, the image processing unit 110 transmits a reconstruction completion notification to the imaging control unit 123. The reconstruction completion notification includes the generated tomosynthesis image, the reconstruction parameters, and the image processing parameters. Upon reception of the reconstruction completion notification, the imaging control unit 123 generates new tomosynthesis image information, and inputs, to the new tomosynthesis image, the tomosynthesis image, the reconstruction parameters, and the image processing parameters included in the reconstruction completion notification. Then, the imaging control unit 123 adds the newly generated tomosynthesis image information out of the imaging technique information included in the scheduled examination information to the imaging technique that has completed the reconstruction. The display control unit 124 hides the progress bar currently displayed in the image display area 1002 displayed on the display unit 109. Then, the display control unit 124 displays a preview of the tomosynthesis image in the image display area 1002 and updates the annotation display. The reconstruction screen 1101 enables the operator to perform an input operation for performing window processing and reproduction processing on the tomosynthesis image and editing the reconstruction parameters.

In response to an input operation for determining reconstruction performed on the operation unit 108, the imaging control unit 123 stores the reconstructed tomosynthesis image. The imaging control unit 123 causes the display control unit 124 to change the display screen on the display unit 109 to the imaging screen 1001, and further adds a captured image thumbnail 1011 of the tomosynthesis image as preview display.

While the reconstructed tomographic image is being displayed as described above, the imaging control unit 123 performs the post-processing on the tomosynthesis image. The post-processing on the tomosynthesis image includes editing a clipped region, displaying tomosynthesis images in parallel (multi-view), a re-imaging instruction, and a reject instruction.

Further, the output specification unit 125 specifies an output target oblique image or tomographic image (coronal image), for example, in response to an input operation performed on the operation unit 108. According to the specification, the information acquisition unit 126 acquires the position and orientation information for specifying the image specified as an output target, and stores the information in the storage unit in association with the image specified as an output target.

In step S611, the imaging control unit 123 determines whether re-imaging is to be performed according to whether re-imaging is specified during the display processing in step S610. When the imaging control unit 123 determines that re-imaging is to be performed (YES in step S611), then in step S604, the imaging control unit 123 selects a similar imaging technique to the imaging information corresponding to the re-imaging, and performs imaging as described above. Since the irradiation condition or the isocenter position may be incorrect, the imaging control unit 123 repeats processing from the center position setting in step S606.

In step S612, the imaging control unit 123 determines whether the next imaging based on the imaging information not captured is to be performed. When there is the imaging information not captured, i.e., when the imaging control unit 123 determines that the next imaging is to be performed (YES in step S612), then in step S606, the imaging control unit 123 selects the imaging information not captured, and performs imaging as described above. The imaging control unit 123 determines whether the next imaging based on the imaging information not captured is to be performed. When there is the imaging information not captured, i.e., when the imaging control unit 123 determines that the next imaging is to be performed (YES in step S612), then in step S606, the imaging control unit 123 selects the imaging information not captured, and performs imaging as described above.

In step S613, the imaging control unit 123 determines whether the end of the examination is specified. The end of the examination is specified by an input operation of pressing an end examination button 1017 on the operation unit 108. When the input operation is performed, the imaging control unit 123 determines that the end of the examination is specified. When an input operation for ending the examination is performed (YES in step S613), the processing proceeds to step S614. On the other hand, when the end of the examination is not specified (NO in step S613), then in step S610, the display control unit 124 displays an imaging screen for the examination that has already been started on the display unit 109.

In step S614, the imaging control unit 123 performs examination end processing. The imaging control unit 123 transmits an examination end notification to the storage unit 403 and the display control unit 124. The examination end notification includes the scheduled examination information. The image output notification includes the scheduled examination information. Upon reception of the examination end notification, the storage unit 403 searches for and acquires the scheduled examination information from among the registered examination information. Then, the storage unit 403 updates the examination status of the acquired examination information to "Examination Ended". Upon reception of the examination end notification, the display control unit 124 changes the screen display on the display unit 109 to the patient information input screen 801. When the operation unit 108 receives an examination suspension instruction, the imaging control unit 123 performs a similar processing flow to the end of the examination. However, the storage unit 403 updates the examination status of the acquired examination information to "Examination Suspended".

In step S615, the file generation unit 404 generates a DICOM image file. The imaging condition selected in step S604 and the information for identifying the position and the orientation of the output target image acquired in step S610 are included in the associated information of the acquired oblique image (and the coronal image).

In step S616, the imaging control unit 123 performs image output processing. The imaging control unit 123 performs the image output processing via the communication circuit 112 according to the end of the examination. For the image specified as an output image in step S610, the communication circuit (output unit) 112 outputs the DICOM image file generated by the file generation unit 404.

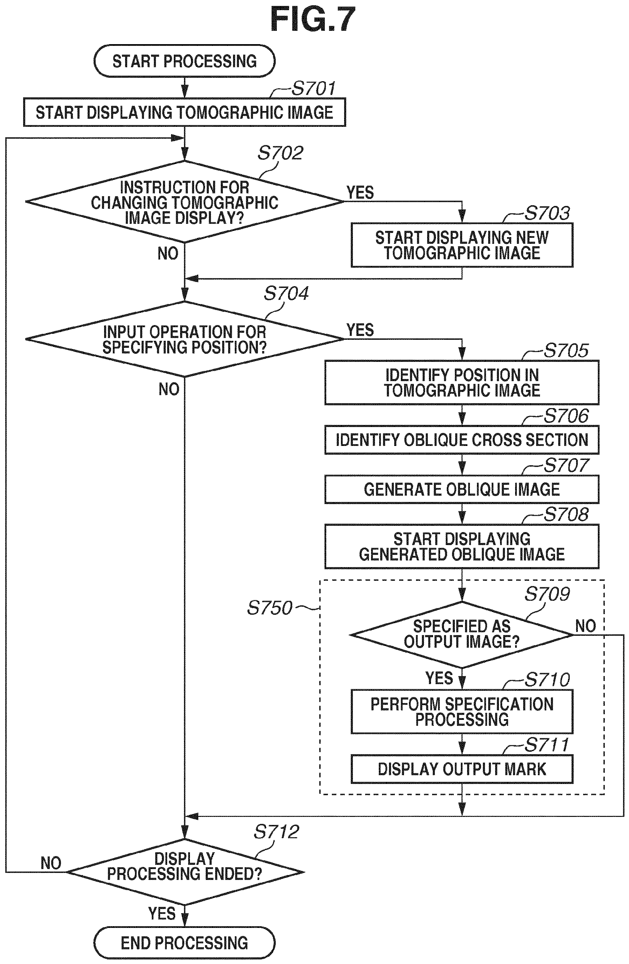

Processing for displaying the tomographic image and processing for specifying the output image in step S610 will be described below with reference to the flowchart illustrated in Fig. 7. The processing illustrated in Fig. 7 relates to processing for displaying the tomographic image (coronal image or oblique image) to be displayed in the image display area 1102 in the reconstruction screen 1101 illustrated in Fig. 11.

In step S701, the display control unit 124 controls the display unit 109 to start displaying the tomographic image of the subject reconstructed from the projection images on the display unit 109. The tomographic image is a coronal image of the subject in the positioning state of the imaging system and the subject illustrated in Figs. 1 to 3. When the tomographic image reconstructed from the projection images includes coronal images of a plurality of sections at different positions in the vertical direction illustrated in Fig. 2. When an input operation for changing tomographic image display is performed on the operation unit 108 (YES in step S702), then in step S703, the display control unit 124 changes tomographic image display in response to the input operation. The imaging control unit 123 determines whether an input operation for changing tomographic image display has been performed, by performing an attempt to detect an input operation of pressing a button on the operation unit 108 corresponding to the display change, and a selection operation on any icon displayed on the display unit 109. When the input operation for changing display is performed (YES in step S702), the processing proceeds to step S703. On the other hand, when the input operation is not performed (NO in step S702), the processing proceeds to step S704. In step S703, the display control unit 124 changes the new tomographic image relating to the change target instead of the tomographic image displayed before changing the tomographic image when the input operation for changing display is performed. For example, in response to an input operation for changing display, the display control unit 124 displays the tomographic image of the cross section adjacent to the cross section of the tomographic image displayed before changing tomographic image display.

In step S704, the imaging control unit 123 determines whether an input operation for specifying a position in the displayed tomographic image is performed. The determination is made by the imaging control unit 123 according to whether an input operation for specifying one point on the tomographic image (coronal image) currently displayed in the image display area 1102 is detected. The identification unit 401 may make the determination in response to an input operation for specifying a position on the tomographic image in a state where a target point button 1118 as a toggle button in the reconstruction screen 1101 is pressed and set to ON.

When the input operation is performed (YES in step S704), the processing proceeds to step S705. On the other hand, when the input operation is not performed (NO in step S704), the processing proceeds to step S712.

In step S705, the identification unit 401 identifies a position corresponding to the input operation as a target point. Information about the identified position is stored in the identification unit 401 or the storage unit 403. When a certain two-dimensional or three-dimensional region is identified through an input operation, the identification unit 401 identifies one point in the region.

In step S706, the image processing unit 111 identifies an oblique cross section which passes through the identified position and the isocenter position. When the identified position is one point outside the isocenter and the imaging system illustrated in Fig. 2 is used (i.e., the isocenter is linear), a cross section is uniquely identified.

In step S707, the image processing unit 111 generates an oblique image based on the cross section. When a tomographic image reconstructed based on the projection images is acquired as voxel data, an oblique image based on the oblique cross section is generated from the voxel data. When a tomographic image is acquired as a plurality of two-dimensional tomographic images, an oblique image will be generated from the two-dimensional tomographic images.

In step S708, the display control unit 124 starts display processing for controlling the display unit 109 to display the oblique image of the cross section passing through the identified position and the isocenter. For example, an oblique image is displayed instead of a tomographic image (coronal image) in the image display area 1102 of the reconstruction screen 1101 illustrated in Fig. 11. Alternatively, when a plurality of display units 109a and 109b is connected to the imaging control apparatus 107, the display control unit 124 displays the generated oblique image on the display unit 109b which is different from the display unit 109a on which the reconstruction screen 1101 including the tomographic image (coronal image) is to be displayed.

In steps S709 to S712, the display control unit 124 performs specification processing for specifying an output target image. Hereinafter, the processing in steps S709 to S712 may be collectively referred to as step S750.

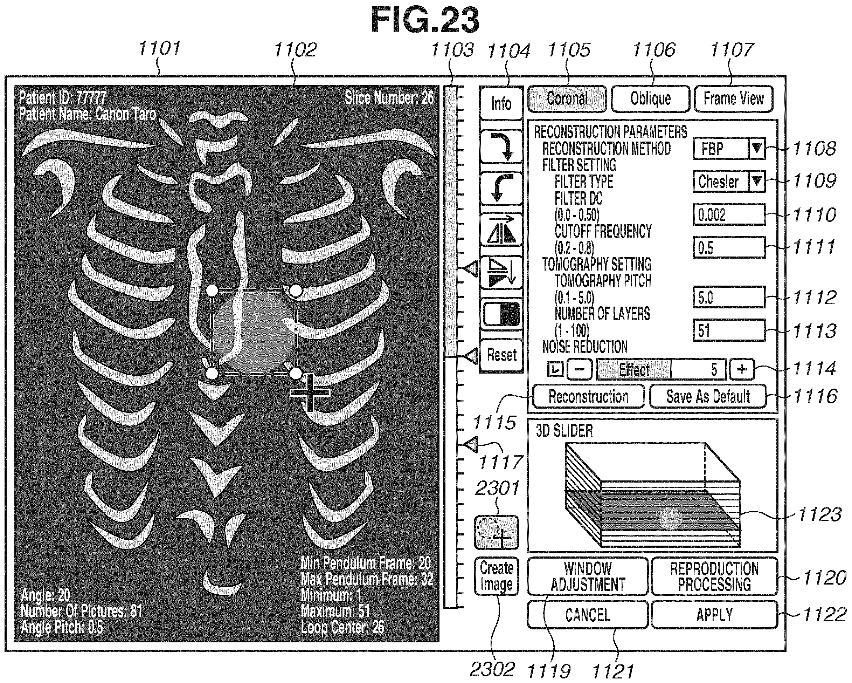

In step S709, the imaging control unit 123 determines whether an input operation for specifying an oblique image or tomographic images (coronal images) as output images has been performed. The input operation for specifying the image as an output image is determined to have been performed, for example, when a button (for specifying an output image) displayed on the operation unit 108 is pressed (see Fig. 23). When the input operation for specifying the image is performed (YES in step S709), the processing proceeds to step S710. On the other hand, when the instruction is not received (NO in step S709), the processing proceeds to step S712.

In step S710, the output specification unit 125 specifies an oblique image and a tomographic image (coronal image) as an output image. The specification target image is an oblique image or a tomographic image displayed on the display unit 109 when the input operation is performed in step S709. Alternatively, when the input operation is performed in step S709 in a state where a target region or a target position is specified in the tomographic image, the output specification unit 125 specifies at least one coronal image and one tomographic image passing through the target region or target position as output targets. In the specification processing, the output specification unit 125 stores the output target image in the storage unit by associating it with the identification information indicating that it is an output target.

In step S711, the display control unit 124 controls the display unit 109 to display at least one output target image, and display an output mark indicating that it is an output target together with the output target image. Subsequently, when the output target image becomes a display target in response to an input operation performed on the operation unit 108, the display control unit 124 attaches an output mark to the output target image.

In step S712, the display control unit 124 determines whether the end of the display processing is specified. The end of the display processing is determined according to whether a reconstruction determination (APPLY) button 1122 in the reconstruction screen 1101 is pressed. When the end of display processing is not specified (NO in step S712), the display control unit 124 will determine whether an input operation for changing display is performed in step S702, whether an input operation for specifying a position is performed in step S704, and whether an input operation for ending display processing is performed in step S712. When an input operation for ending display processing is performed (YES in step S712), the display control unit 124 ends the display processing.

Examples of screens displayed on the display unit 109 by the display control unit 124 according to the present embodiment will be described below with reference to Fig. 8 to 19.

An example of the patient information input screen 801 displayed in step S601 illustrated in Fig. 6 will be described below with reference to Fig. 8. The patient information input screen 801 is a screen for inputting information of an examination target patient. The patient information input screen 801 includes a patient information input area 802, a patient information list 803, a patient information determination (APPLY) button 804, a patient information display area 805, an examination information display area 806, and a start examination button (START EXAMINATION) 807. The patient information input area 802 is an area for inputting and selecting the value of each item included in the patient information. The patient information list 803 displays a list of patient information relating to examinations performed in the past. Each column of the patient information list 803 displays one item included in the patient information. In the patient information list 803, the patient information for each person is displayed in each row. When arbitrary patient information is selected in the patient information list 803, the selected patient information is input in each input area in the patient information input area 802. The patient information determination button 804 is used to determine the value input in the patient information input area 802 as patient information. When the operator presses this button, the display control unit 124 checks whether values are input in mandatory input items or corrects values conforming to criteria are input as input items. When there is no problem, the input values are determined as patient information. The patient information display area 805 displays the determined patient information. The value of each item is not displayed before the patient information is determined, and displayed when the patient information is determined. The examination information display area 806 displays input examination information. The examination information includes information for specifying an examination, such as examination ID, reference doctor name, radiogram interpretation doctor name, examination descriptions, facility name. The examination information also includes an imaging technique selected as an imaging schedule. At least one imaging technique can be selected for each examination. The examination information display area 806 includes an area for displaying each item of the examination information and an area for displaying a selected imaging technique. The value of each item is not displayed before the examination information is input. Similarly, the imaging technique is not displayed before it is selected. These two pieces of information are displayed when the examination information is input and when an imaging technique is selected, respectively. Further, for each imaging, a plurality of examinations can be performed at one time. In this case, the examination information display area 806 is displayed in an arranged form for the number of examinations. The "Start Examination" button 807 issues an instruction for starting an examination. When the operator presses this button, the imaging control unit 123 checks whether the patient information and the examination information are input, and checks whether at least one imaging technique is selected for each examination. When there is no problem, the imaging control unit 123 performs the examination start processing. When there is at least one examination for which no imaging technique is selected, the imaging technique selection screen 901 is displayed.

Fig. 9 illustrates an example of the imaging technique selection screen 901 displayed in step S602 illustrated in Fig. 6. The imaging technique selection screen 901 is used to select a scheduled imaging technique in the scheduled examination information. The imaging technique selection screen 901 includes the imaging technique display area 902, imaging technique buttons 903, an patient information display area 904, an examination information display area 905, a selected imaging technique button 906, and a start examination (START EXAMINATION) button 907. The imaging technique display area 902 displays each of imaging techniques stored in the storage unit 403 by using the imaging technique buttons 903. The button display position can be changed arbitrarily. When the display of imaging techniques does not fit into one page, imaging techniques can be displayed in a plurality of pages. In this case, the display page can be changed by a page change instruction. An imaging technique button 903 is displayed for each imaging technique stored in the storage unit 403. Each imaging technique button 903 displays the name of the imaging technique and the name of the sensor to be used. When the operator presses this button, the currently selected examination is determined as an imaging schedule. The patient information display area 904 displays the determined patient information. The examination information display area 905 displays input examination information. The selected imaging technique button 906 displays the imaging technique button 903 selected in the imaging technique display area 902. Since at least one imaging technique can be selected for an examination, the selected imaging technique button 906 is added to the end of the examination information display area 905 each time an imaging technique button 903 is selected. The start examination button 807 issues an instruction for starting an examination. When the operator presses this button, the display control unit 124 determines whether the patient information and the examination information are input and checks whether at least one imaging technique is selected for each examination. When there is no problem, the display control unit 124 performs the examination start processing. When the examination start processing is performed, the screen changes to the imaging screen 1001. When there is at least one examination for which no imaging technique is selected, the display control unit 124 prompts the operator to select an imaging technique, and does not perform screen transition. The imaging technique selection screen 901 having the above-described configuration is displayed.

Fig. 10 illustrates an example of the imaging screen 1001 displayed in step S603 illustrated in Fig. 6. The imaging screen 1001 includes the image display area 1002, a status display area 1003, a single view button 1004, a multi view button 1005, a frame view button 1006, a patient information display area 1007, an examination information display area 1008, an imaging technique display area 1009, a reconstruction button 1010, a captured image thumbnail 1011, an imaging schedule thumbnail 1012, a window level editing box 1013, a window width editing box 1014, a suspend examination button 1015, an output image button 1016, an end examination button 1017, an annotation display (Info) button 1018, a clockwise rotation button 1019, a counterclockwise rotation button 1020, a horizontal inversion button 1021, a vertical inversion button 1022, a black-and-white inversion button 1023, an L mark arrangement button 1024, an R mark arrangement button 1025, a clipping setting (Crop) button 1026, a mask processing (Mask) button 1027, a re-imaging button 1028, a reject button 1029, an undo button 1030, and a reset button 1031. The image display area 1002 displays a preview of a captured image after still-image capturing or a tomosynthesis image after the reconstruction processing. During moving image capturing, the image display area 1002 displays a preview of the captured moving image in real time. When preview selection is changed after image capturing, a captured image selected to be previewed is previewed. Further, according to settings, the patient information, the examination information, and the irradiation condition are displayed as annotations. No image is displayed in the initial state immediately after an examination is started. The status display area 1003 displays statuses notified from the X-ray control unit 105 and the X-ray detector 104 in different colors and texts to make it easier for the operator to distinguish the statuses. Upon reception of a status notification from the X-ray control unit 105 and the X-ray detector 104 via the communication circuit 112, the imaging control unit 123 determines the display contents based on the combination of the statuses of the X-ray control unit 105 and the X-ray detector 104, and transmits a status display change instruction to the display control unit 124. For example, when the X-ray control unit 105 is unable to perform X-ray irradiation or the X-ray detector 104 is unable to perform X-ray detection, the X-ray control unit 105 displays "Not Ready" on the sensor status. Further, when the X-ray control unit 105 is able to perform X-ray irradiation and the X-ray detector 104 is able to perform X-ray detection, the X-ray control unit 105 displays "Ready" on the sensor status with the background color easily distinguishable from that for "Not Ready". The single view button 1004 is used to select single view for displaying one frame of the image selected to be previewed in the image display area 1002. In a case of images of a plurality of frames, it is possible to display other frames and reproduce a moving image through keyboard or mouse operations during preview display. The multi view button 1005 is used to select multi-view for displaying a group of images captured within the examination currently being executed in parallel in a plurality of lattice-shaped division display areas in the image display area 1002. Before two or more images are captured in the currently executed examination, the multi view button 1005 is disabled and the multi-view display is not possible. The frame view button 1006 is used to select frame view for displaying in parallel a group of frame images of a moving image selected to be previewed in a plurality of lattice-shaped division display areas in the image display area 1002. When the image selected to be previewed is not a moving image, the frame view button 1006 is disabled and the frame view display is not possible. The patient information display area 1007 displays the patient information such as the patient name and the patient ID. The examination information display area 1008 displays the examination information such as the examination ID and examination descriptions. Imaging techniques selected for an examination are displayed in an arranged form in the imaging technique display area 1009. The imaging technique display area 1009 includes the reconstruction button 1010, the captured image thumbnail 1011, and the imaging schedule thumbnail 1012. The imaging technique display area 1009 displays imaging technique information such as imaging technique names, and the captured image thumbnail 1011 for all of captured images. In the initial state immediately after starting an examination, the captured image thumbnail 1011 is not displayed since imaging has not yet been performed. The reconstruction button 1010 issues an instruction for performing the reconstruction processing on a tomosynthesis imaging technique including the image currently selected to be previewed. The reconstruction button 1010 is not displayed and the display area is truncated for imaging techniques other than tomosynthesis imaging techniques. Further, when a plurality of tomosynthesis imaging techniques is displayed, the reconstruction button 1010 is disabled for other than tomosynthesis imaging techniques including the image currently selected to be previewed. Issuing an instruction by using the reconstruction button 1010 enables performing reconstruction processing on a tomosynthesis imaging technique that has once completed the reconstruction processing. The captured image thumbnail 1011 displays a thumbnail image, an imaging type mark, a similar mark 2301, and a reject mark 2601 corresponding to each captured image. The imaging type mark enables distinguishing the imaging type: still image, fluoroscopic image, cineradiographic image, and tomosynthesis image. For example, "C" indicates a cineradiographic image and "T" indicates a tomosynthesis image. However, the imaging type is not limited thereto as long as the imaging type is distinguishable. The preview display changes by selecting the captured image thumbnail 1011. The imaging technique display area 1009 selected for the next scheduled irradiation displays the imaging schedule thumbnail 1012 in which a position where a thumbnail will be added in the next irradiation is left blank. When the scheduled irradiation selection state is canceled, the imaging schedule thumbnail 1012 is hidden. The window level editing box 1013 and the window width editing box 1014 are used to edit the window level and the window width of the image currently selected to be previewed, respectively. By changing the value displayed in the editing box or dragging with the mouse on the image display area 1002, editing is applied to the image currently being previewed. The suspend examination button 1015 issues an instruction for suspending the examination currently being executed. The imaging control unit 123 performs examination suspending processing in response to the pressing of the button. The output image button 1016 issues an instruction for outputting the captured image included in the examination currently being executed. When the image output is specified, an image is output as in step 616 described above. The end examination button 1017 issues an instruction for ending the examination currently being executed. The imaging control unit 123 performs examination end processing in response to the pressing of the button. The annotation display (Info) button 1018 displays or hides the annotations displayed in the image display area 1002. The clockwise rotation button 1019 is used to rotate clockwise the captured image currently being previewed. The counterclockwise rotation button 1020 is used to rotate counterclockwise the captured image currently being previewed. The horizontal inversion button 1021 is used to perform horizontal inversion on the captured image currently being previewed. The vertical inversion button 1022 is used to perform vertical inversion on the captured image currently being previewed. The black-and-white inversion button 1023 is used to reverses the window value of the captured image currently being previewed. The L mark arrangement button 1024 is used to arrange a laterality marker "L" on the captured image currently being previewed. The L mark arrangement button 1024 can be turned ON or OFF. Turning the L mark arrangement button 1024 ON displays "L" and turning it OFF hides "L". The R mark arrangement button 1025 is used to arrange a laterality marker "R" on the captured image currently being previewed. The R mark arrangement button 1025 can be turned ON or OFF. Turning the R mark arrangement button 1025 ON displays "R" and turning it OFF hides "R". The clipping setting (Crop) button 1026 issues an instruction for setting clipping of the target region in the captured image currently being previewed. The mask processing (mask) button 1027 issues an instruction for performing mask processing on the captured image currently being previewed. The re-imaging button 1028 issues an instruction for performing re-imaging on an imaging technique including the image currently selected to be previewed. The re-imaging in this case refers to performing reject processing on an image specified for re-imaging and then performing processing for adding the same imaging technique as a new one. The reject button 1029 issues an instruction for performing reject processing on the image currently selected to be previewed. When the reject processing is performed, a reject setting included in the image information is set to ON. The undo button 1030 issues an instruction for performing undo processing for undoing one piece of processing that has been performed on the image currently selected to be previewed, from the latest one backward. The reset button 1031 issues an instruction for performing reset processing for undoing all pieces of processing that have been performed on the image currently selected to be previewed, and returning the image to the state immediately after imaging. The imaging screen 1001 having the above-described configuration is displayed.

Fig. 11 illustrates an example of the reconstruction screen 1101 displayed in step S609 illustrated in Fig. 6. The reconstruction screen 1101 includes an image display area 1102, a frame specification slider 1103, an image operation tool bar 1104, a coronal cross section display (Coronal) button 1105, an oblique cross section display (Oblique) button 1106, a frame view button 1107, a reconstruction method selection drop-down list 1108, the reconstruction filter type selection drop-down list 1109, a reconstruction filter DC editing box 1110, a cutoff frequency editing box 1111, the tomographic pitch editing box 1112, a number of layers editing box 1113, a noise reduction processing editing box 1114, a reconstruction processing button 1115, a default setting (Save As Default) button 1116, a frame reproduction range setting marker 1117, a target point button 1118, a window adjustment display button 1119, a reproduction processing button 1120, a reconstruction cancel (CANCEL) button 1121, a reconstruction determination button 1122, and a 3D slider 1123. The image display area 1102 displays the tomosynthesis image after reconstruction processing as a preview. During execution of the reconstruction processing, a progress bar for notifying the operator of execution of the reconstruction processing is displayed, and a tomosynthesis image is displayed at the same time as the completion of the reconstruction processing. The frame specification slider 1103 is used to confirm and change a frame image displayed in the tomosynthesis image currently being previewed. At the same time as the tomosynthesis image preview, an equally divided scale is displayed for all of effective frames of the tomosynthesis image currently being previewed, from the top to the bottom ends of the frame specification slider 1103. Performing control to specify only effective frames enables avoiding the risk of displaying invalid frames. When the operator performs a selection or a drag operation on the frame specification slider 1103, a frame having the number corresponding to the selected scale is displayed in the image display area 1102. In the image operation tool bar 1104, controls for specifying processing to be performed on the tomosynthesis image currently being previewed, are arranged. The arranged controls are similar to controls 918 to 931 arranged in the imaging screen 1001. The coronal cross section display button 1105 specifies tomosynthesis image display based on a coronal cross section in the image display area 1102. The oblique cross section display button 1106 specifies tomosynthesis image display based on an oblique cross section in the image display area 1102. The frame view button 1107 is used to select frame view for displaying in parallel a group of frame images of a tomosynthesis image currently being previewed in a plurality of lattice-shaped division display areas in the image display area 1002. During oblique cross section display, the frame view button 1107 is disabled and the frame view display is not possible. The reconstruction method selection drop-down list 1108 is a control for selecting a reconstruction method such as the Filter Back Projection (FBP) method and the Shift-and-Add method. The reconstruction filter type selection drop-down list 1109 is a control for selecting the filter type to be used when the reconstruction processing is performed. The reconstruction filter DC editing box 1110 is a control for editing a DC parameter of the filter to be used when the reconstruction processing is performed. The cutoff frequency editing box 1111 is a control for editing the cutoff frequency of the filter to be used when the reconstruction processing is performed. The tomographic pitch editing box 1112 is a control for editing the thickness between frames when the reconstruction processing is performed. The number of layers editing box 1113 is a control for editing the total number of frames when the reconstruction processing is performed. The noise reduction processing editing box 1114 is a control for specifying whether to apply noise reduction processing when the reconstruction processing is performed and for editing the degree of influence when the noise reduction processing is applied. The reconstruction processing (Reconstruction) button 1115 issues an instruction for performing the reconstruction processing. When the operator presses the reconstruction processing button, the reconstruction processing is performed again by using the currently input reconstruction parameters. In this case, the same projection images as those for the tomosynthesis image currently being previewed are used. The default setting button 1116 issues an instruction for changing the default reconstruction parameters for the tomosynthesis imaging technique currently being previewed. When the operator presses the default setting button 1116, the imaging control unit 406 transmits the currently displayed reconstruction parameters, and a reconstruction parameter change notification to the examination implementation 407. The imaging control unit 123 updates the reconstruction parameters of the tomosynthesis imaging technique to be subjected to the reconstruction parameters, and transmits a "registration and update" processing request to the storage unit 403. The frame reproduction range setting marker 1117 is a control for specifying a reproduction range at the time of specified range reciprocal reproduction. The frame reproduction range setting marker 1117 includes three different markers for specifying a minimum frame number, a center frame number, and a maximum frame number, respectively. By moving each marker, a range from the specified minimum frame number to the specified maximum frame number is set as a reproduction range. The target point button 1118 specifies a target point that passes through the oblique image. The window adjustment display button 1119 is used to display and hide a window adjustment control. When the window adjustment display button 1119 is set to ON, the window adjustment control is displayed in the display area of the 3D slider 1123. When the window adjustment display button 1119 is set to OFF, the window adjustment control is hidden and the 3D slider 1123 is displayed. The reproduction processing button 1120 is used to display and hide a reproduction processing control. When the reproduction processing button 1120 is set to ON, the reproduction processing control is displayed in the display area of the 3D slider 1123. When the reproduction processing button 1120 is set to OFF, the reproduction processing control is hidden and the 3D slider 1123 is displayed. The reconstruction cancel button 1121 issues an instruction for cancelling the tomosynthesis image currently being previewed. When the reconstruction cancel is specified, step S609 is completed and the screen changes to the imaging screen 1001 without storing the tomosynthesis image and the image information. In the imaging screen 1001, the image previewed before the reconstruction screen display is continuously selected to be previewed. The reconstruction determination button 1122 issues an instruction for storing and determining the tomosynthesis image currently being previewed. When storing and determining of the tomosynthesis image are specified, the tomosynthesis image currently being previewed is stored in the HDD 504. Then, step S608 is completed and the screen changes to the imaging screen 1001. The 3D slider 1123 is a control for three-dimensionally displaying frames of the generated tomosynthesis image in a pseudo way, and specifying a frame to be displayed. On the 3D slider 1123, a ruled line indicates the relative positional relation between frames of the tomosynthesis image, and a reduced image is displayed at a position having the same frame number as the displayed frame image. Selecting the ruled line on the 3D slider 1123 or dragging it with the mouse enables easily changing the frame to be displayed. The ruled line is displayed on the 3D slider 1123 for the number of effective frames of the tomosynthesis image. In preview display, in association with the editing of the tomographic pitch or the number of layers, the positional relation of frames of each tomosynthesis image after the reconstruction processing is superimposed onto the current state. This enables easily grasping changes in the thickness when the operator changes the tomographic pitch and the number of layers. The reconstruction screen 1101 having the above-described configuration is displayed.

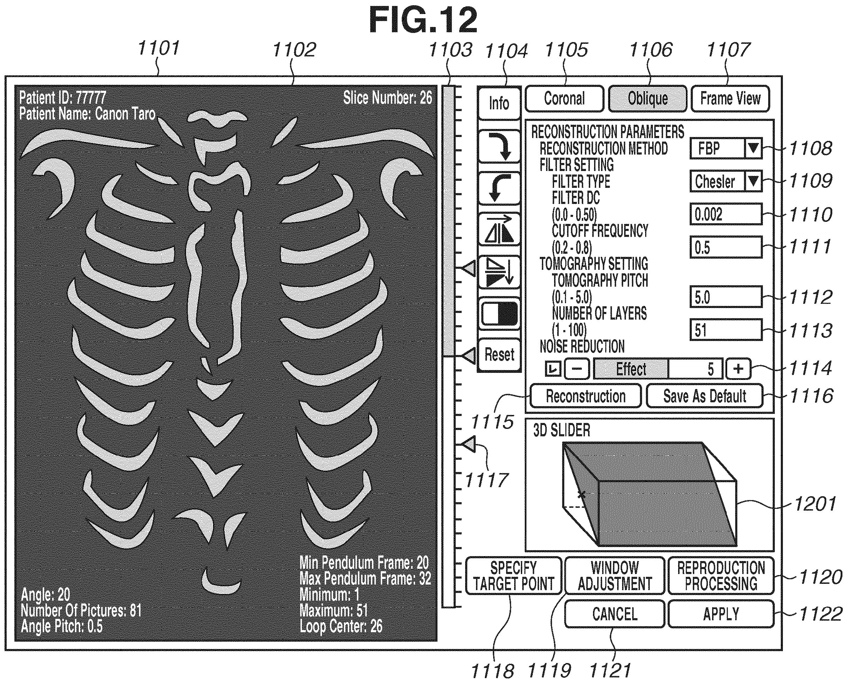

Fig. 12 illustrates the reconstruction screen 1101 for the oblique cross section display in step S609 illustrated in Fig. 6 according to an embodiment. When the operator presses the oblique cross section display button 1106, the cross section of the tomosynthesis image displayed in the image display area 1102 changes from a coronal cross section to an oblique cross section. When the operator presses the coronal cross section display button 1105, the cross section of the tomosynthesis image displayed in the image display area 1102 changes from an oblique cross section to a coronal cross section. During oblique cross section display, the frame specification by the frame specification slider 1103 and the reproduction specification from the reproduction processing unit 1901 are ignored. Further, the frame view button 1107 is disabled and the frame view display is not possible. During oblique cross section display, instead of the regular 3D slider 1123, a 3D slider 1201 for editing the oblique angle is displayed. The oblique angle is changed corresponding to the editing of the display angle of the frame image currently displayed on the 3D slider 1201 for editing the oblique angle. In association with the oblique angle edited by the 3D slider 1201 for editing the oblique angle, the oblique angle of the tomosynthesis image previewed in the image display area 1102 is also changed. The reconstruction screen 1101 for the oblique cross section display having the above-described configuration is displayed.

Figs. 13 to 16 illustrate in detail an example of a screen transition from the coronal cross section display to the oblique cross section display.

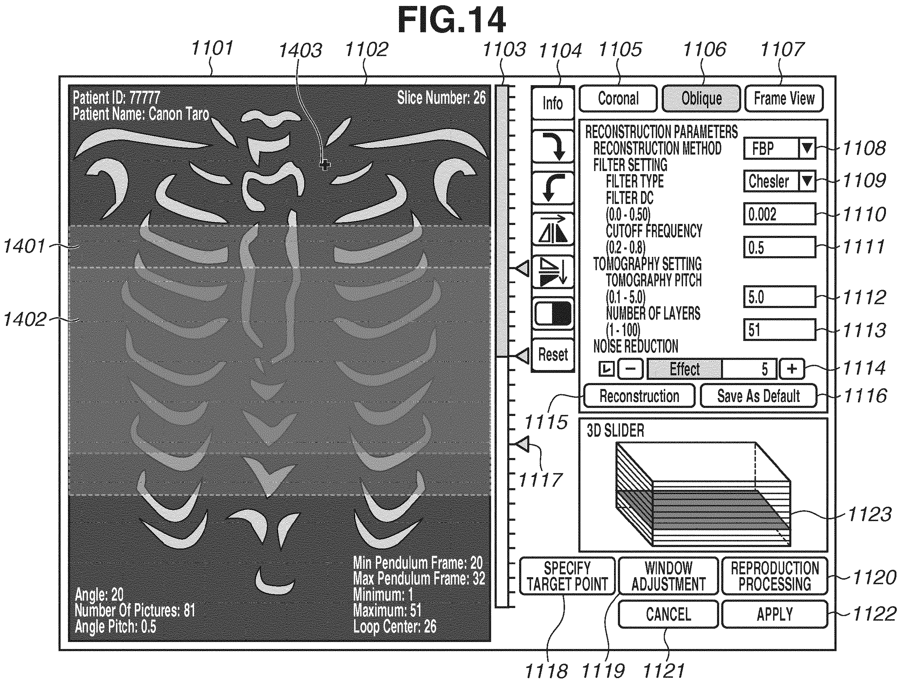

Fig. 13 illustrates an example of a pop-up screen 1301 displayed when the operator presses the oblique cross section display button 1106 during the coronal cross section display illustrated in Fig. 11. The pop-up screen 1301 is displayed in the imaging screen 1001. The pop-up screen 1301 displays a message for making an inquiry to the operator about whether to specify a target point on the current coronal image, together with a Yes button 1302 and a No button 1303. When the operator presses the Yes button 1302, the screen changes to the one illustrated in Fig. 14. On the other hand, when the operator presses the No button 1303, the screen changes to the oblique cross section display illustrated in Fig. 12. In addition, when the operator presses the target point button 1118 during the coronal cross section display illustrated in Fig. 11, an icon may change to a position input state. Further, when the operator presses the oblique cross section display button 1106 after specifying one target point, the reconstruction screen 1101 for the oblique cross section display illustrated in Fig. 12 may be displayed without displaying the pop-up screen illustrated in Fig. 13. In a case where the upper-limit number of target points is set to 1 and stored in the identification unit 401, an icon may change to the position input state when the operator presses the target point button 1118. Further, when one target point is specified, the reconstruction screen 1101 for the oblique cross section display illustrated in Fig. 12 may be displayed without pressing the oblique cross section display button 1106.

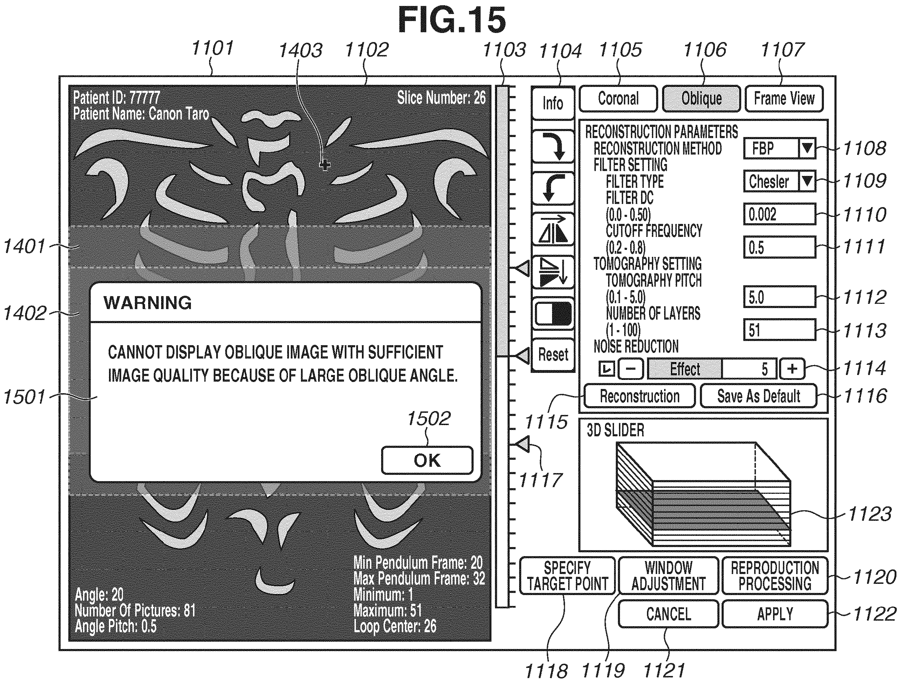

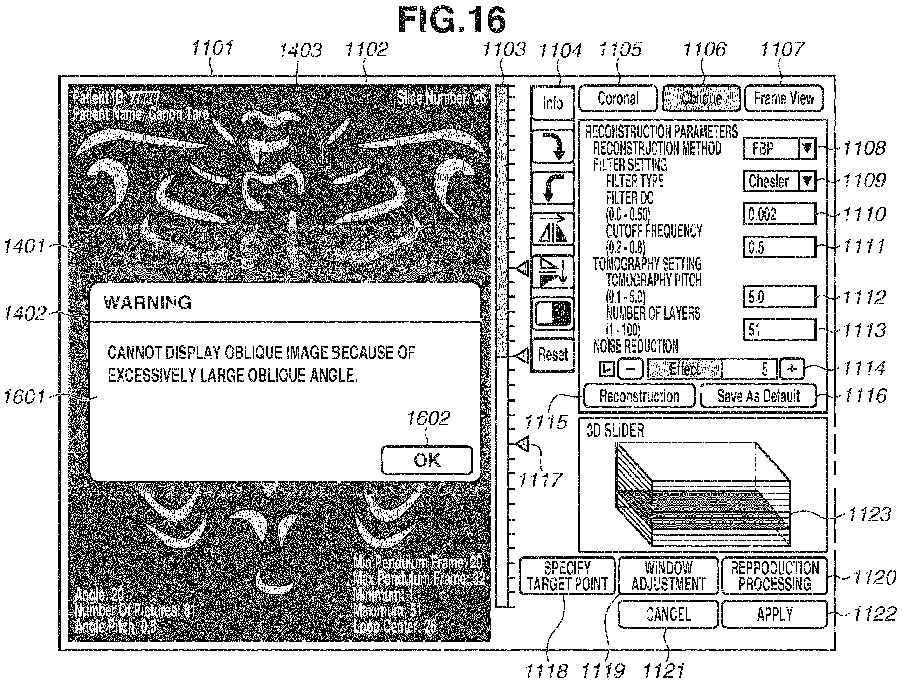

Fig. 14 illustrate an example of a range relating to the identification by the identification unit 401, displayed in the tomographic image by the display control unit 124. The image display area 1102 includes a warning display region display area 1401 corresponding to the range relating to the identification, and a generation prohibition region display area 1402 indicating a range identifiable by the identification unit 401. Within the range, a mouse icon 1403 changes to a point specification shape and moves to the outside of the warning display region display area 1401. The warning display region display area 1401 is a rectangular area of which the top and the bottom sides respectively have two different linear coordinates calculated from a first threshold value of the oblique angle stored in the storage unit 403. By superimposing a rectangle having a predetermined color and a predetermined transparency onto the area, the warning display region display area 1401 makes it harder to view the coronal image than in areas where the rectangular area does not exist. The generation prohibition region display area 1402 is a rectangular area of which the top and the bottom sides respectively have two different linear coordinates calculated from a second threshold value of the oblique angle stored in the storage unit 403. The generation prohibition region display area 1402 makes it harder to view the coronal image than the warning display region display area 1401 does. The above-described display control is achieved by decreasing the transparency of the rectangle to be superimposed onto the area. When the operator clicks the outside of the warning display region display area 1401 in the image display area 1101, an oblique angle made by the clicked coordinates and the isocenter line is calculated. Then, an oblique image making the calculated oblique angle is generated, and then the screen changes to the oblique cross section display illustrated in Fig. 12. When the operator clicks the inside of the warning display region display area 1401 and the outside of the generation prohibition region display area 1402, the display control unit 124 changes the screen displayed on the display unit 109 to the screen illustrated in Fig. 15, and displays a message including a predetermined warning on the display unit 109. When the operator clicks the inside of the generation prohibition region display area 1402, the display control unit 124 changes the screen displayed on the display unit 109 to the screen illustrated in Fig. 16, and restricts the oblique image display of a cross section including the position outside the above-described range.

Fig. 15 illustrates an example of a pop-up screen 1501 displayed when the operator clicks the inside of the warning display region display area 1401 illustrated in Fig. 14 and the outside of the generation prohibition region display area 1402 illustrated in Fig. 15. The pop-up screen 1501 is displayed in the imaging screen 1001. The pop-up screen 1501 displays a message indicating that an oblique image cannot be displayed with sufficient image quality because of a large oblique angle, together with an OK button 1502. When the operator presses the OK button 1502, the pop-up screen 1501 is closed. Then, an oblique angle made by the clicked coordinates and the isocenter line is calculated, an oblique image making the calculated oblique angle is generated, and the screen changes to the oblique cross section display illustrated in Fig. 12.

Fig. 16 illustrates an example of a pop-up screen 1601 displayed when the operator clicks the inside of the generation prohibition region display area 1402 illustrated in Fig. 14. The pop-up screen 1601 is displayed in the imaging screen 1001. The pop-up screen 1601 displays a message indicating that an oblique image cannot be displayed because of an excessively large oblique angle, and recommending the operator to perform re-imaging with other modality such as CT and MRI, together with an OK button 1602. When the operator presses the OK button 1602, the pop-up screen 1601 is closed.

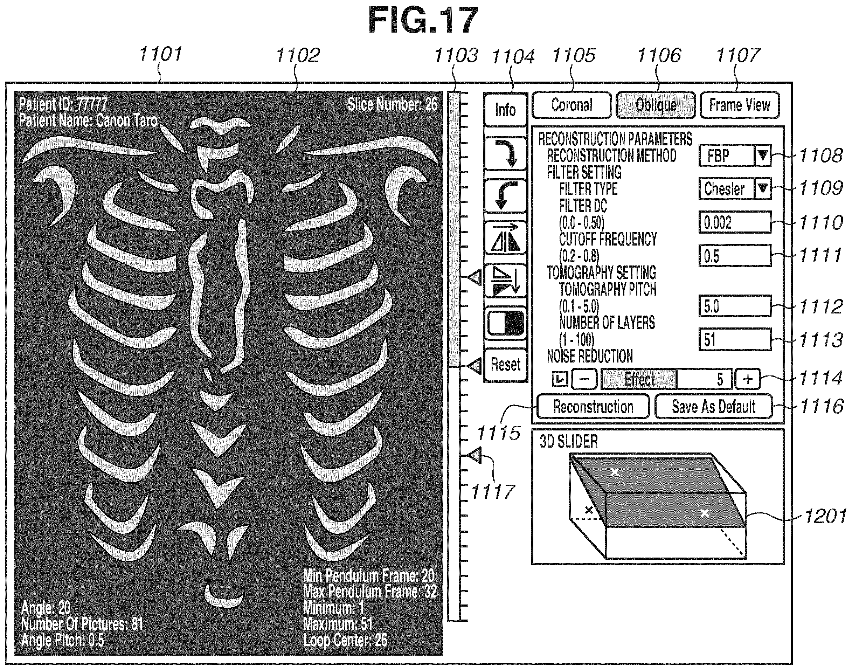

Referring to Figs. 17 to 20, an example of a screen transition from the coronal cross section display to the oblique cross section display according to the present embodiment, will be described in detail.

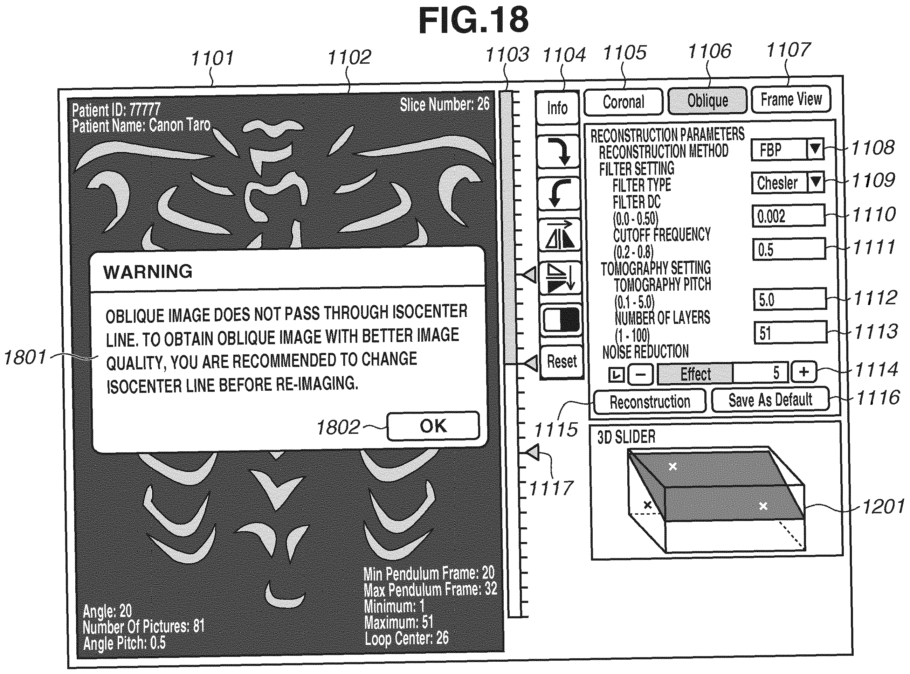

When the operator presses the oblique cross section display button 1106 after specifying two target points by using the target point button 1118 illustrated in Fig. 11, the reconstruction screen 1101 for the oblique cross section display illustrated in Fig. 17 is displayed. The oblique image displayed in Fig. 17 is an image of a cross section passing through the specified two points and parallel to the isocenter line. Before the oblique cross section display illustrated in Fig. 17 is made or when shifting to other than the oblique cross section display, a message "Oblique Image Does Not Pass Through Isocenter Line. To Obtain Oblique Image with Better Image Quality, You Are Recommended To Change Isocenter Line Before Re-imaging." as illustrated in Fig. 18 may be displayed. Further, in a case where the upper-limit number of target points is set to 2 and stored in identification unit 401, when the operator specifies two target points by using the target point button 1118, the reconstruction screen 1101 for the oblique cross section display illustrated in Fig. 17 may be displayed without pressing the oblique cross section display button 1106.

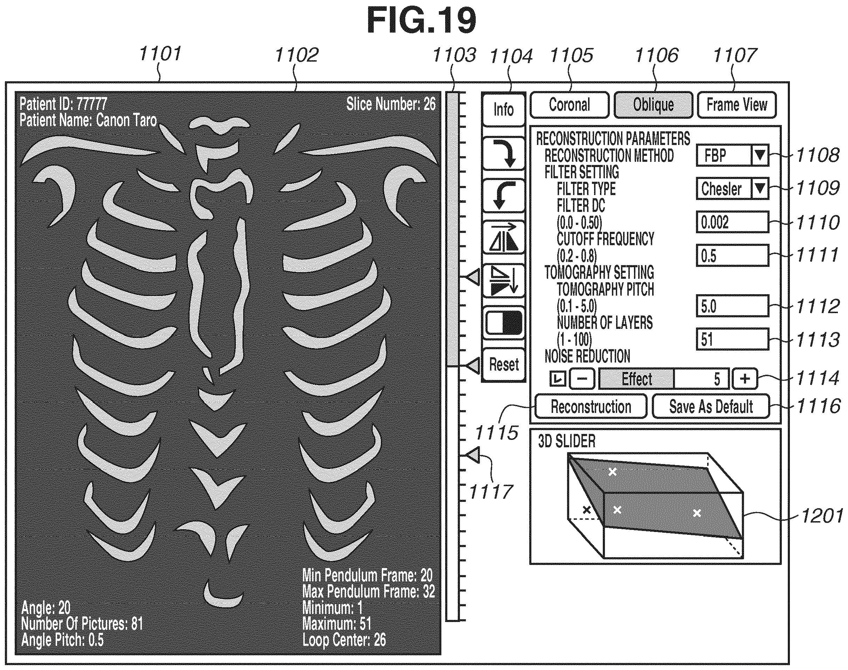

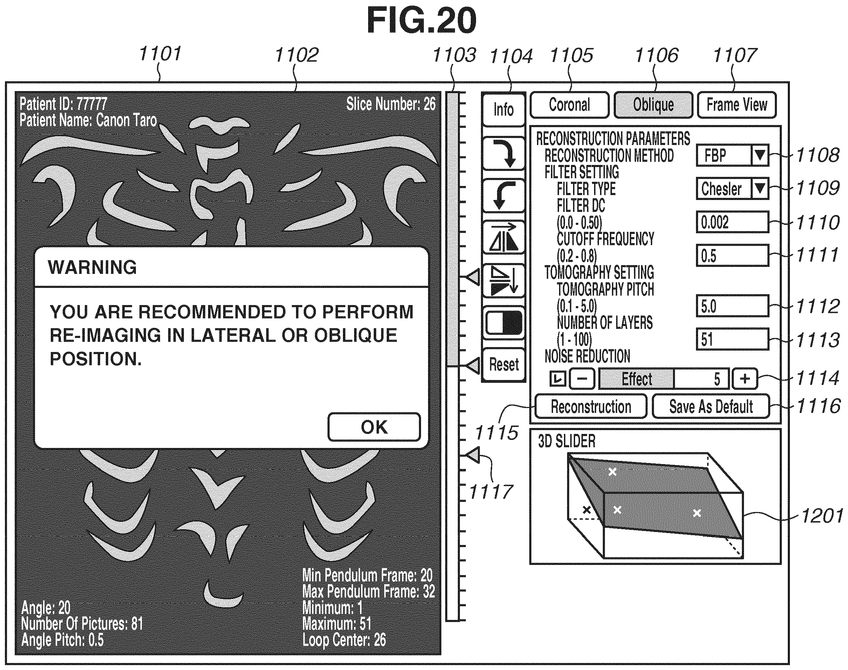

When the operator presses the oblique cross section display button 1106 after specifying three target points by using the target point button 1118 illustrated in Fig. 11, the reconstruction screen 1101 for the oblique cross section display illustrated in Fig. 19 is displayed. The oblique image displayed in Fig. 19 is an image of a cross section passing through the three specified points. In a case where the angle of the oblique image of the cross section is close to a sagittal cross section, before the oblique cross section display illustrated in Fig. 19 is made, or when shifting to other than the oblique cross section display, a message "You Are Recommended To Perform Re-imaging in Lateral or Oblique Position." as illustrated in Fig. 20 may be displayed. In a case where the upper-limit number of target points is set to 3 and stored in the identification unit 401, when the operator specifies three target points by using the target point button 1118, the reconstruction screen 1101 for the oblique cross section display illustrated in Fig. 19 may be displayed without pressing the oblique cross section display button 1106.

When the operator presses the oblique cross section display button 1106 after specifying three target points by using the target point button 1118 illustrated in Fig. 11, the reconstruction screen 1101 for the oblique cross section display illustrated in Fig. 19 is displayed. The oblique image displayed in Fig. 19 is an image of a cross section passing through the three specified points. In a case where the angle of the oblique image of the cross section is close to a sagittal cross section, before the oblique cross section display illustrated in Fig. 19 is made, or when shifting to other than the oblique cross section display, a message "You Are Recommended To Perform Re-imaging in Lateral or Oblique Position." as illustrated in Fig. 20 may be displayed. In a case where the upper-limit number of target points is set to 3 and stored in the identification unit 401, when the operator specifies three target points by using the target point button 1118, the reconstruction screen 1101 for the oblique cross section display illustrated in Fig. 19 may be displayed without pressing the oblique cross section display button 1106.

When the operator specifies four or more target points by using the target point button 1118, a combination of three suitable target points or less may be selected by the selection unit 402. As a method for selecting three target points or less, it is desirable to decrease the oblique angle which is an angle made by the oblique image passing through three points or less and the coronal image. Further, all of the specified target points may be selected once or a plurality of times. More specifically, for example, when four target points (A to D) are specified and all of the target points are selected once, there are four different combinations of three points and one point: ((A, B, C), D), ((A, B, D), C), ((A, C, D), B), and ((B, C, D), A). There are three different combinations of two points and two points: ((A, B), (C, D)), ((A, C), (B, D)), and ((A, D), (B, C)). It may also be possible to calculate each oblique angle and select a combination in which the sum of the oblique angles is minimized. Alternatively, for example, 10 different combinations of oblique images passing through two to three points are displayed in a list form, and the display may be changed through an operator's selection. When the combinations are displayed in a list form, the oblique angle of each oblique image may be displayed, and oblique angles may be sorted in order of angle.

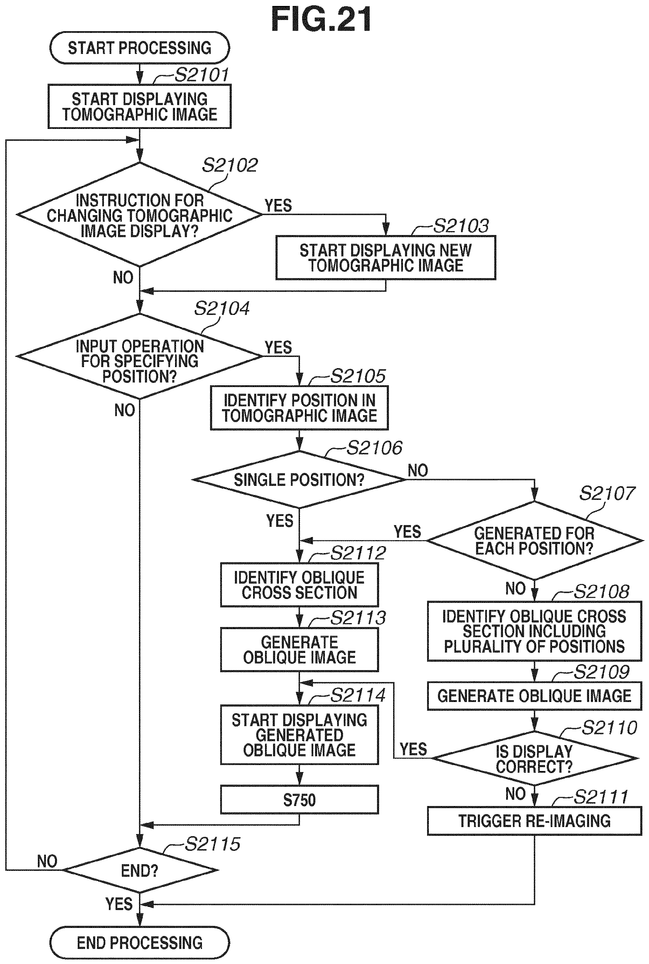

Referring to Fig. 21, an example of display processing according to another embodiment, will be described. The example of the display processing differs from the display processing illustrated in Fig. 7 in that the following pieces of processing (1) to (4) are additionally performed.
(1) In step S2106, the display control unit 124 determines whether a plurality of positions is specified.
(2) In step S2107, the display control unit 124 determines whether to acquire an oblique image for each of a plurality of the specified positions or to acquire an oblique image including a plurality of the specified positions.
(3) In step S2110, the display control unit 124 determines whether the generated oblique image is allowed to be displayed.
(4) In step S2111, the display control unit 124 triggers re-imaging. Although, in the present embodiment, all of processing (2) to (4) are considered to be performed, the display control unit 124 may perform only one of these pieces of processing or only a combination of a part of them. Alternatively, the display control unit 124 may perform only processing (3) and (4) without performing (1).

Processing in steps S2101 to S2105 is similar to the processing in steps S701 to S705 illustrated in Fig. 7, and descriptions thereof will be omitted. Likewise, processing in steps S2112 to S2114, S750, and S2115 is similar to the processing in steps S706 to S712, and descriptions thereof will be omitted.

In step S2106, the identification unit 401 determines whether a single position or a plurality of positions is specified. In step S2104, the identification unit 401 determines whether an input operation for specifying a position is performed, by determining whether the target point button 1118 is pressed to be set to ON and then set to OFF, and positions corresponding to all of input operations are specified. The identification unit 401 determines whether a single position or a plurality of positions is specified based on the number of the specified positions counted by the imaging control unit 123. When only one position is specified (YES in step S2106), the processing proceeds to step S2112. On the other hand, when a plurality of positions is specified (NO in step S2106), the processing proceeds to S2107.

In step S2107, the imaging control unit 123 determines whether to generate an oblique image for each of a plurality of the specified positions or to generate an oblique image including a plurality of the specified positions. The imaging control unit 123 makes the determination by determining whether an input operation for acquiring which of the oblique images has been performed. When two positions are specified, the imaging control unit 123 determines whether to generate two different oblique images corresponding to the two positions or to generate one oblique image including the two positions. When three positions are specified, the imaging control unit 123 determines whether to generate three oblique images corresponding to the three position or to generate one oblique image including the three positions. When the imaging control unit 123 determines to generate an oblique image for each of a plurality of the specified positions (YES in step S2107), the processing proceeds to step S2112. Then, the imaging control unit 123 performs steps S2112 and S2113 for each individual position. When the imaging control unit 123 determines to generate an oblique image including a plurality of the specified positions (NO in step S2107), the processing proceeds to step S2108.

When three positions are specified, the imaging control unit 123 may determine whether to generate two different oblique images, i.e., an oblique image including any two positions and an oblique image including the remaining one position. In this case, the imaging control unit 123 performs processing in step S2108 and subsequent steps for the two positions, and performs processing in steps S2112 and S2113 for the one remaining position. The specified positions are selected, for example, by the selection unit 402. The imaging control unit 123 may perform the two pieces of processing in parallel or on a time sharing manner.

Alternatively, the imaging control unit 123 may generate only an oblique image including a part of positions selected by the selection unit 402 out of a plurality of the specified positions. The imaging control unit 123 determines whether to perform processing in step S2112 and subsequent steps or to perform processing in step S2108 and subsequent steps depending on whether a plurality of positions is specified and on whether a plurality of oblique images each corresponding to an individual position is to be generated.

In step S2108, the image processing unit 110 identifies an oblique cross section including a plurality of the specified positions. In step S2109, the image processing unit 110 generates an oblique image of the specified oblique cross section. The processing thereof is performed as described above.

In step S2110, the imaging control unit 123 determines whether the generated oblique image satisfies a display target criterion. In the determination processing, various methods such as a method of comparing image qualities and a method of using orientation information of the oblique image can be used. When the imaging control unit 123 determines that the oblique image satisfies the display target criterion (YES in step S2110), the processing proceeds to step S2114. On the other hand, when the imaging control unit 123 determines that the oblique image does not satisfy the criterion (NO in step S2110), the processing proceeds to step S2111.

In still another embodiment, when the image data of the oblique image is not directly used, the imaging control unit 123 performs the determination processing in step S2110 before the oblique image generation processing in step S2109. This enables preventing an unnecessary oblique image from being generated. For example, when determining whether the oblique image satisfies the criterion based on the orientation of the oblique cross section, the imaging control unit 123 is able to perform the determination processing based on the information of the orientation of the identified oblique cross section after processing for specifying an oblique cross section in step S2108.

In still another embodiment, the imaging control unit 123 may combine the processing for determining whether the oblique image is a display target in step S2110 and the processing for determining whether to generate an oblique image for each of a plurality of the specified positions in step S2107. For example, when the imaging control unit 123 determines that the oblique image including a plurality of the specified positions does not satisfy the criterion, the imaging control unit 123determines to generate a plurality of oblique images respectively including a plurality of positions (YES in step S2107).

In step S2111, the imaging control unit 123 triggers re-imaging. Triggering re-imaging refers to outputting an instruction signal for starting processing for generating an imaging condition for re-imaging and processing for displaying a message indicating that re-imaging should be performed. For example, the imaging control unit 123 generates an imaging condition in response to the triggering of re-imaging. The display control unit 124 controls the display unit 109 to display the above-described message and an imaging condition for re-imaging. The image processing unit 110 generates the above-described pseudo scout image. This enables suitably supporting re-imaging.

In step S2114, when a plurality of oblique images are generated and a plurality of the oblique images are a display target, for example, the display control unit 124 controls the display unit 109 to display a plurality of the oblique images in an arranged form. Then, in step S750, the output specification unit 125 performs processing for specifying output target images.

In the above-described embodiments, the imaging control unit 123 may perform processing for determining whether to display an oblique image including only one specified position.

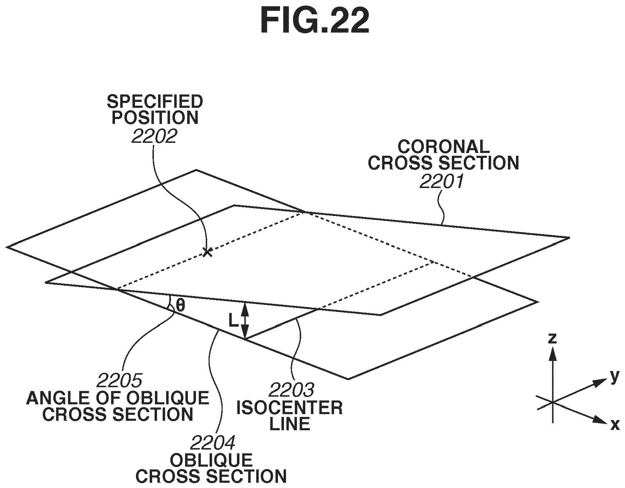

A positional relation between a coronal cross section and an oblique cross section according to the present embodiment will be described below with reference to Fig. 22. Referring to Fig. 22, it is assumed that the detection surface of the X-ray detector 106 is the xy plane, the X-ray generation apparatus 102 moves in the xz plane along the x-axis direction.

A coronal cross section 2201 is a cross section located at a position distant from an isocenter line 2203 by a distance L in the z direction. For example, the display control unit 124 displays a coronal image of the coronal cross section 2201 on the display unit 109. The identification unit 401 identifies a position 2202 on the coronal image based on an input operation performed on the operation unit 109 or a result of image analysis by the image processing unit 110. The image processing unit 110 identifies an oblique cross section 2204 including the identified position 2202 and the isocenter line 2203, and generates an oblique image of the oblique cross section 2204. The oblique cross section 2204 intersects with the coronal cross section 2201 at an angle 2205. The display control unit 124 controls the display unit 109 to display an oblique image of the oblique cross section 2204. The identified position 2202 can be observed by using the coronal image of the coronal cross section 2201 and the oblique image of the oblique cross section 2204, enabling quickly acquiring an image suitable for diagnosis.

According to the above-described embodiments, it becomes possible to specify a plurality of target points from a coronal image having clearest target points, and immediately display an oblique image including the target points, remarkably improving operability. Further, if the angle of the oblique image or the distance from the coronal image passing through the rotation center line exceeds a fixed threshold value, the operator is notified of the situation. This enables restricting generation and display of an oblique image exceeding an identical or other threshold value, and hence enables reducing the possibility of misdiagnosis.

Referring to Fig. 23, an example of a GUI for selecting a target region in response to an input operation performed on the operation unit 108, will be described. For components assigned the same reference numerals as those illustrated in Fig. 11, descriptions will be omitted. The reconstruction screen 1101 displays a target region button 2301 and an output image generation button 2302.

When the operator presses the target region button 2301, the identification unit 401 identifies the target region. When the operator presses the output image generation button 2302, the output specification unit 125 specifies as an output target a predetermined reconstructed image including the target region. The file generation unit 404 generates a DICOM image of the specified reconstructed image. When the operator presses the output image generation button 2302 without specifying a target region, the output specification unit 125 specifies the coronal image or the oblique image currently displayed in the image display area 1102 as an output target. The file generation unit 404 generates a DICOM file of the image. An output target can be added a plurality of times.

When the operator presses the target region button 2301, the mouse pointer changes to a cross pointer to make it easier to suitably specify a position in the image. When the operator clicks any desired position in the image display area 1102 in this state, a circular object is displayed around the clicked position. The region surrounded by the circular object is a target region. The operator is able to move the target region by dragging the circular object through an input operation. Further, a dashed-line rectangle is displayed around the target region. The operator is able to change the radius of the target region by dragging the corners of the rectangle. The position and radius of a sphere indicating the target region are restricted so that the sphere does not protrude from the inside of the volume of the three-dimensional reconstructed image. The 3D slider 1123 three-dimensionally displays the target region as a sphere. The three-dimensionally displayed target region is updated in association with the movement and size change of the target region in the above-described image display area 1102. When the operator presses the output image generation button 2302 with the target region being displayed, the output specification unit 125 adds the coronal image and the oblique image surrounding the target region as storage target reconstructed images.

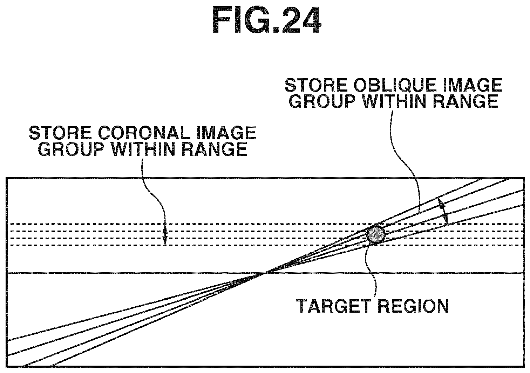

Referring to Fig. 24, an output target tomographic image when a target region is specified, is described. Fig. 24 illustrates a three-dimensional reconstructed image when viewed from an extension of the isocenter line in a direction perpendicular to the isocenter line. When the operator presses the output image generation button 2302 in the GUI illustrated in Fig. 23, the output specification unit 125 specifies as an output target the coronal image of each of a plurality of division planes between the top and the bottom tangent planes in the target region parallel to the bottom plane, as one image data group. In this case, the captured image thumbnail 1011 stores a coronal image having the coronal plane Y coordinates closest to the center of the target region out of the image data group. The output specification unit 125 further specifies as an output target an oblique image of each of a plurality of division planes in an angle made between the tangent planes of two points in the target region passing through the isocenter line, as one image data group. In this case, an oblique image closest to the plane which equally divides the angle made between the tangent planes of two points in the target region passing through the isocenter line is stored in the captured image thumbnail 1011. In still another embodiment, the output specification unit 125 may specify only an oblique image (when an oblique image is displayed) or only a coronal image (when a coronal image is displayed) as an output target.

An example of a storage format of a reconstructed image will be described below. When a reconstructed image in tomosynthesis imaging is transmitted to an external apparatus, it is necessary to store the reconstructed image in a state of indicating that reconstructed image is an image (secondary capture image) secondarily acquired through X-ray imaging a plurality of times. In DICOM, a data format "DIGITAL X-RAY IMAGE INFORMATION OBJECT" for transmitting one X-ray still image and a data format "X-RAY RF IMAGE INFORMATION OBJECT" for transmitting X-ray images of a plurality of frames are defined. These data formats include an Image Type tag for indicating an image acquisition method. Therefore, indicating a secondary capture image by using the tag enables transmitting an image as a secondary capture image. In DICOM, further, a data format "SECONDARY CAPTURE IMAGE INFORMATION" for storing a secondary capture image is defined. In recent years, even in a case of one image, it is recommended to transmit the image with the number of frames being set to 1 using a data format "MULTI-FRAME GRAYSCALE WORD SC IMAGE IOD MODULES" similar to images of a plurality of frames, out of the data format "SECONDARY CAPTURE IMAGE INFORMATION". Even when a target region is specified and images of a plurality of frames are used as one set of storage data or when one tomographic image is used as one DICOM image, using the data format "MULTI-FRAME GRAYSCALE WORD SC IMAGE IOD MODULES" enables storing the data in a similar format by changing the number of frames. Using the data formats "X-RAY RF IMAGE INFORMATION OBJECT" and "MULTI-FRAME GRAYSCALE WORD SC IMAGE IOD MODULES" enables transmitting an image data group in the same target region as one set of image data having a plurality of frames. Further, appending data (such as the oblique angle, the coronal plane height, parameters at the time of reconstruction, and imaging condition) as parameters makes it easier to implement an observation environment, for example, when re-imaging is performed. Further, when pieces of data such as an examined portion, center coordinates of the target region, and an examination date are appended as parameters, the operator is able to recognize an oblique image group and a coronal image group for the same target region as one group.

According to the above-described embodiments, it becomes possible to output only an oblique image as a diagnosis target, and also to alleviate the load on communication as well as the capacity stress on the PACS server.

In the above-described embodiments, when volume data is acquired from a plurality of projection images, at least one oblique image, at least one three orthogonal cross-sectional image, or partial volume data, which are smaller than the volume data, is output. However, the embodiments of the present invention are not limited thereto. Apart from the viewpoint of the decrease in the number of data items, when an observation target region is large or when the observation target region is to be observed by using more cross sections, the communication circuit (output unit) 122 may output volume data as it is. In this case, when volume data is output, by including at least one of an observation angle considered to be the most suitable and information of the position and the orientation of a cross section considered to be the most suitable as associated information, it becomes possible to suitably support diagnosis based on the volume data. Alternatively, instead of volume data, by outputting to the PACS 115 a DICOM image in which the above-described associated information is included in a plurality of projection images acquired by the X-ray detector (image capturing unit) 106 through tomosynthesis imaging, it becomes possible to perform the reconstruction processing which best suits diagnostic needs via the PACS 115 or a workstation. Therefore, it becomes very convenient.

Although, in the above-described embodiments, an imaging apparatus performs tomosynthesis imaging with X-rays, an apparatus for performing tomography with radiation other than X-rays or tomography using a medium other than radiation may be used.

Although, in the above-described embodiments, the imaging control apparatus 107 is a single apparatus, a control system including a plurality of the information processing apparatuses may be used in another embodiment. In this case, each of a plurality of the information processing apparatuses has a communication circuit enabling communication with each other. One of a plurality of the information processing apparatuses is able to function as the image processing unit 110, and another apparatus is able to function as the imaging control unit 123. These information processing apparatuses do not need to exist in the same hospital or in the same country as long as they are capable of communicating with each other at a predetermined communication rate. In such a control system, for example, the image processing unit 110 may be a server apparatus or a server group shared by a plurality of control systems.

Embodiments of the present invention include an embodiment in which software (program) for implementing the above-described functions is supplied to a system or apparatus, and a computer of the system or apparatus reads and executes the supplied program. Further, an operating system (OS) operating on the computer may perform a part or whole of actual processing based on instructions included in the program read by the computer, and the functions of the above-described embodiments may be implemented through the processing.

Embodiments of the present invention also include an embodiment in which the above-described embodiments are suitably combined.

While the present invention has been described with reference to embodiments, it is to be understood that the invention is not limited to the disclosed embodiments. The scope of the following claims is to be accorded the broadest interpretation so as to encompass all such modifications and equivalent structures and functions.

## Claims

1. An apparatus (101, 107) for controlling tomosynthesis imaging using an imaging device including a sensor (106) in which pixels are two-dimensionally arranged, the apparatus comprising:
at least one communication circuit (112, 506);
at least one processor (501); and
at least one memory (502, 503, 504) including a program that includes instructions to be executed by the at least one processor to implement processing, the at least one processor configured to:
set an imaging condition of tomosynthesis imaging;
output a signal, for controlling the sensor (106), to the imaging device via the at least one communication circuit (112, 506) based on the set imaging condition;
cause a display device (109) to display at least one tomographic image including an oblique image based on projection images obtained from the sensor (106) controlled by the signal;
designate the displayed oblique image;
obtain information of a position and an orientation of the designated oblique image;
output the designated oblique image as an image obtained by the tomosynthesis imaging; and
output the designated oblique image, via the at least one communication circuit (112, 506), as a DICOM image including information of the set imaging condition and information for identifying the position and the orientation of the oblique image in associated information.

2. The apparatus for controlling tomosynthesis imaging according to claim 1, wherein in designating the displayed oblique image, an oblique image based on an oblique cross section passing through a position or region in the tomographic image, is designated.

3. The apparatus for controlling tomosynthesis imaging according to claim 1, wherein in designating the displayed oblique image, a plurality of the oblique images are designated.

4. The apparatus for controlling tomosynthesis imaging according to claim 3, wherein the output DICOM image includes identification information common to a plurality of the oblique images in the associated information of each DICOM image for each of a plurality of the oblique images.

5. The apparatus for controlling tomosynthesis imaging according to claim 2, wherein the output DICOM image includes information of the position or region in the associated information of each DICOM image for each of a plurality of the oblique images output.

6. The apparatus for controlling tomosynthesis imaging according to claim 1, wherein in designating the displayed oblique image, the oblique image, and at least one tomographic image of at least any one of three orthogonal cross sections including a coronal cross section, an axial cross section, and a sagittal cross section, are designated.

7. The apparatus for controlling tomosynthesis imaging according to claim 6, wherein the output DICOM image includes information of the identification information common to the tomographic image and the oblique image in the associated information of each DICOM image for the tomographic image of the three orthogonal cross sections and the oblique image.

8. The apparatus for controlling tomosynthesis imaging according to claim 6,
wherein in designating the displayed oblique image, an oblique image based on at least one cross section passing through a position or region in the tomographic image, identified according to an input operation from an operation means (108), and at least one tomographic image of at least any one of the three orthogonal cross sections including a coronal cross section, an axial cross section, and a sagittal cross section, are designated, and
wherein the output DICOM image includes information of the position or region in the associated information of each DICOM image for the tomographic image of the three orthogonal cross sections and the oblique image.

9. The apparatus for controlling tomosynthesis imaging according to claim 1, wherein in designating the displayed oblique image, an oblique image based on at least one cross section passing through a three-dimensional region in the tomographic image, identified according to an input operation from an operation means, is designated, and
wherein in the outputting of the DICOM image, partial volume data of the three-dimensional region is output in addition to the DICOM image of the designated oblique image.

10. The apparatus for controlling tomosynthesis imaging according to claim 1, wherein the at least one processor (501) is further configured to:
generate DICOM image data including the designated oblique image as image data, and including the information of the set imaging condition and the information for identifying the position and the orientation of the oblique image, as associated information.

11. The apparatus for controlling tomosynthesis imaging according to claim 10, wherein in generating DICOM image data, a DICOM image of the designated oblique image, including first identification information as associated information is generated, and a DICOM image of the tomographic image of three orthogonal cross sections, including second identification information different from the first identification information as associated information, is generated.

12. An image management apparatus (114, 115, 116, 117) for receiving a DICOM image output from the apparatus (101, 107) for controlling tomosynthesis imaging according to claim 11, the image management apparatus comprising:
at least one processor; and
at least one memory including a program including instructions to be executed by the at least one processor to implement processing, the at least one processor configured to:
make setting for differentiating a storage period or a storage image format between the DICOM image to be provided with the first identification information and the DICOM image to be provided with the second identification information.

13. A method for controlling tomosynthesis imaging using an imaging device including a sensor (106) in which a plurality of pixels are two-dimensionally arranged, the method comprising:
setting an imaging condition of tomosynthesis imaging;
transmitting a signal, to the imaging device, for controlling the sensor (106) based on the set imaging condition;
causing a display device (109) to display a tomographic image, including an oblique image, based on a plurality of projection images obtained by the sensor (106) ;
designating the oblique image;
obtaining information of a position and an orientation of the designated oblique image; and
outputting a DICOM image including the designated oblique image as an image obtained in the tomosynthesis imaging, and including information of the set imaging condition and information for identifying the position and the orientation of the oblique image, in associated information.

14. A computer-readable recording medium storing a program that when executed on a computer causes the computer to execute the control method according to claim 13.

## Patentansprüche

1. Vorrichtung (101, 107) zum Steuern von Tomosynthesebildgebung unter Verwendung einer Bildgebungsvorrichtung, die einen Sensor (106) beinhaltet, in dem Pixel zweidimensional angeordnet sind, wobei die Vorrichtung umfasst:
wenigstens eine Kommunikationsschaltung (112, 506);
wenigstens einen Prozessor (501); sowie
wenigstens einen Speicher (502, 503, 504) einschließlich eines Programms, das Anweisungen beinhaltet, die durch den wenigstens einen Prozessor ausgeführt werden sollen, um eine Verarbeitung zu implementieren, wobei der wenigstens eine Prozessor konfiguriert ist:
um eine Bildgebungsbedingung einer Tomosynthesebildgebung einzustellen;
um basierend auf der eingestellten Bildgebungsbedingung über die wenigstens eine Kommunikationsschaltung (112, 506) ein Signal zum Steuern des Sensors (106) an die Bildgebungsvorrichtung auszugeben;
um zu veranlassen, dass eine Anzeigevorrichtung (109) wenigstens ein tomografisches Bild, das ein Schrägbild beinhaltet, basierend auf Projektionsbildern anzeigt, die von dem durch das Signal gesteuerten Sensor (106) erhalten wurden;
um das angezeigte Schrägbild zu bestimmen;
um Information über eine Position und eine Orientierung des bestimmten Schrägbilds zu erhalten;
um das bestimmte Schrägbild als ein durch die Tomosynthesebildgebung erhaltenes Bild auszugeben; und
um das bestimmte Schrägbild über die wenigstens eine Kommunikationsschaltung (112, 506) als ein DICOM-Bild auszugeben, das Information über die eingestellte Bildgebungsbedingung und Information zum Identifizieren der Position und der Orientierung des Schrägbilds in verknüpfter Information beinhaltet.

2. Vorrichtung zum Steuern von Tomosynthesebildgebung nach Anspruch 1,
wobei beim Bestimmen des angezeigten Schrägbilds ein Schrägbild basierend auf einem eine Position oder Region im tomografischen Bild durchlaufenden schrägen Querschnitt bestimmt wird.

3. Vorrichtung zum Steuern von Tomosynthesebildgebung nach Anspruch 1,
wobei beim Bestimmen des angezeigten Schrägbilds mehrere der Schrägbilder bestimmt werden.

4. Vorrichtung zum Steuern von Tomosynthesebildgebung nach Anspruch 3,
wobei das ausgegebene DICOM-Bild Identifikationsinformation, die mehreren der Schrägbilder gemeinsam ist, in der verknüpften Information von jedem DICOM-Bild für ein jeweiliges von mehreren der Schrägbilder beinhaltet.

5. Vorrichtung zum Steuern von Tomosynthesebildgebung nach Anspruch 2,
wobei das ausgegebene DICOM-Bild Information über die Position oder Region in der verknüpften Information von jedem DICOM-Bild für jedes von mehreren der Schrägbilder, die ausgegeben wurden, beinhaltet.

6. Vorrichtung zum Steuern von Tomosynthesebildgebung nach Anspruch 1,
wobei beim Bestimmen des angezeigten Schrägbilds das Schrägbild sowie wenigstens ein tomografisches Bild von wenigstens einem von drei orthogonalen Querschnitten, die einen frontalen Querschnitt, einen transversalen Querschnitt und einen sagittalen Querschnitt beinhalten, bestimmt werden.

7. Vorrichtung zum Steuern von Tomosynthesebildgebung nach Anspruch 6,
wobei das ausgegebene DICOM-Bild Information der Identifikationsinformation, die dem tomografischen Bild und dem Schrägbild gemeinsam ist, in der verknüpften Information von jedem DICOM-Bild für das tomografische Bild der drei orthogonalen Querschnitte und das Schrägbild beinhaltet.

8. Vorrichtung zum Steuern von Tomosynthesebildgebung nach Anspruch 6,
wobei beim Bestimmen des angezeigten Schrägbilds ein Schrägbild, das auf wenigstens einem eine Position oder Region im tomografischen Bild durchlaufenden Querschnitt basiert und das gemäß einer Eingabeoperation von einer Bedienungseinrichtung (108) identifiziert wurde, sowie wenigstens ein tomografisches Bild von wenigstens einem der drei orthogonalen Querschnitte, die einen frontalen Querschnitt, einen transversalen Querschnitt und einen sagittalen Querschnitt beinhalten, bestimmt werden, und
wobei das ausgegebene DICOM-Bild Information über die Position oder Region in der verknüpften Information von jedem DICOM-Bild für das tomografische Bild der drei orthogonalen Querschnitte und das Schrägbild beinhaltet.

9. Vorrichtung zum Steuern von Tomosynthesebildgebung nach Anspruch 1,
wobei beim Bestimmen des angezeigten Schrägbilds ein Schrägbild, das auf wenigstens einem eine dreidimensionale Region im tomografischen Bild durchlaufenden Querschnitt basiert und das gemäß einer Eingabeoperation von einer Bedienungseinrichtung identifiziert wurde, bestimmt wird, und
wobei beim Ausgeben des DICOM-Bilds Teilvolumendaten der dreidimensionalen Region zusätzlich zum DICOM-Bild des bestimmten Schrägbilds ausgegeben werden.

10. Vorrichtung zum Steuern von Tomosynthesebildgebung nach Anspruch 1,
wobei der wenigstens eine Prozessor (501) weiterhin konfiguriert ist, um DICOM-Bilddaten zu generieren, die das bestimmte Schrägbild als Bilddaten beinhalten, und die die Information über die eingestellte Bildgebungsbedingung und die Information zum Identifizieren der Position und der Orientierung des Schrägbilds als verknüpfte Information beinhalten.

11. Vorrichtung zum Steuern von Tomosynthesebildgebung nach Anspruch 10,
wobei beim Generieren von DICOM-Bilddaten ein DICOM-Bild des bestimmten Schrägbilds generiert wird, das erste Identifikationsinformation als verknüpfte Information beinhaltet, und ein DICOM-Bild des tomografischen Bilds von drei orthogonalen Querschnitten generiert wird, das von der ersten Identifikationsinformation verschiedene zweite Identifikationsinformation als verknüpfte Information beinhaltet.

12. Bildverwaltungsvorrichtung (114, 115, 116, 117) zum Empfangen eines DICOM-Bilds, das von der Vorrichtung (101, 107) zum Steuern von Tomosynthesebildgebung nach Anspruch 11 ausgegeben wurde, wobei die Bildverwaltungsvorrichtung umfasst:
wenigstens einen Prozessor; sowie
wenigstens einen Speicher einschließlich eines Programms, das Anweisungen beinhaltet, die durch den wenigstens einen Prozessor ausgeführt werden sollen, um eine Verarbeitung zu implementieren, wobei der wenigstens eine Prozessor konfiguriert ist,
um eine Einstellung vorzunehmen, um einen Speicherzeitraum oder ein Speicherbildformat zwischen dem DICOM-Bild, das mit der ersten Identifikationsinformation versehen werden soll, und dem DICOM-Bild, das mit der zweiten Identifikationsinformation versehen werden soll, zu unterscheiden.

13. Verfahren zum Steuern von Tomosynthesebildgebung unter Verwendung einer Bildgebungsvorrichtung, die einen Sensor (106) beinhaltet, in dem mehrere Pixel zweidimensional angeordnet sind, wobei das Verfahren umfasst:
Einstellen einer Bildgebungsbedingung einer Tomosynthesebildgebung;
Übertragen eines Signals an die Bildgebungsvorrichtung zum Steuern des Sensors (106) basierend auf der eingestellten Bildgebungsbedingung;
Veranlassen, dass eine Anzeigevorrichtung (109) ein tomografisches Bild, das ein Schrägbild beinhaltet, basierend auf mehreren durch den Sensor (106) erhalten Projektionsbildern anzeigt;
Bestimmen des Schrägbilds;
Erhalten von Information über eine Position und eine Orientierung des bestimmten Schrägbilds; sowie
Ausgeben eines DICOM-Bilds, das das bestimmte Schrägbild als ein in der Tomosynthesebildgebung erhaltenes Bild beinhaltet, und das Information über die eingestellte Bildgebungsbedingung und Information zum Identifizieren der Position und der Orientierung des Schrägbilds in verknüpfter Information beinhaltet.

14. Computerlesbares Aufzeichnungsmedium, das ein Programm speichert, welches bei Ausführung auf einem Computer den Computer veranlasst, das Steuerverfahren nach Anspruch 13 durchzuführen.

## Revendications

1. Appareil (101, 107) destiné à commander une formation d'images de tomosynthèse au moyen d'un dispositif de formation d'images comprenant un capteur (106) dans lequel des pixels sont disposés de manière bidimensionnelle, l'appareil comprenant :
au moins un circuit de communication (112, 506) ;
au moins un processeur (501) ; et
au moins une mémoire (502, 503, 504) comprenant un programme qui comprend des instructions à exécuter par l'au moins un processeur pour mettre en oeuvre un traitement, l'au moins un processeur étant configuré pour :
définir une condition de formation d'images de formation d'images de tomosynthèse ;
délivrer en sortie un signal, pour commander le capteur (106), au dispositif de formation d'images par le biais de l'au moins un circuit de communication (112, 506) sur la base de la condition de formation d'images définie ;
amener un dispositif d'affichage (109) à afficher au moins une image tomographique comprenant une image oblique, sur la base des images de projection obtenues à partir du capteur (106) commandé par le signal ;
désigner l'image oblique affichée ;
obtenir des informations d'une position et d'une orientation de l'image oblique désignée ;
délivrer en sortie l'image oblique désignée en tant qu'image obtenue par la formation d'images de tomosynthèse ; et
délivrer en sortie l'image oblique désignée, par le biais de l'au moins un circuit de communication (112, 506), en tant qu'image DICOM comprenant des informations concernant la condition de formation d'images définie et des informations permettant d'identifier la position et l'orientation de l'image oblique dans des informations associées.

2. Appareil destiné à commander une formation d'images de tomosynthèse selon la revendication 1, dans lequel, lors de la désignation de l'image oblique affichée, une image oblique, basée sur une coupe transversale oblique passant par une position ou une région dans l'image tomographique, est désignée.

3. Appareil destiné à commander une formation d'images de tomosynthèse selon la revendication 1, dans lequel, lors de la désignation de l'image oblique affichée, une pluralité des images obliques sont désignées.

4. Appareil destiné à commander une formation d'images de tomosynthèse selon la revendication 3, dans lequel l'image DICOM délivrée en sortie comprend des informations d'identification communes à une pluralité des images obliques dans les informations associées de chaque image DICOM pour chacune d'une pluralité des images obliques.

5. Appareil destiné à commander une formation d'images de tomosynthèse selon la revendication 2, dans lequel l'image DICOM délivrée en sortie comprend des informations concernant la position ou la région dans les informations associées de chaque image DICOM pour chacune de la pluralité des images obliques délivrées en sortie.

6. Appareil destiné à commander une formation d'images de tomosynthèse selon la revendication 1, dans lequel, lors de la désignation de l'image oblique affichée, l'image oblique et au moins une image tomographique d'au moins l'une quelconque de trois coupes transversales orthogonales, comprenant une coupe transversale coronale, une coupe transversale axiale et une coupe transversale sagittale, sont désignées.

7. Appareil destiné à commander une formation d'images de tomosynthèse selon la revendication 6, dans lequel l'image DICOM délivrée en sortie comprend des informations concernant les informations d'identification communes à l'image tomographique et à l'image oblique dans les informations associées de chaque image DICOM de l'image tomographique des trois coupes transversales orthogonales et de l'image oblique.

8. Appareil destiné à commander une formation d'images de tomosynthèse selon la revendication 6,
dans lequel, lors de la désignation de l'image oblique affichée, une image oblique basée sur au moins une coupe transversale passant par une position ou une région dans l'image tomographique, identifiée conformément à une opération d'entrée à partir d'un moyen d'exploitation (108), et au moins une image tomographique d'au moins l'une quelconque des trois coupes transversales orthogonales, comprenant une coupe transversale coronale, une coupe transversale axiale et une coupe transversale sagittale, sont désignées, et
dans lequel l'image DICOM délivrée en sortie comprend des informations concernant la position ou la région dans les informations associées de chaque image DICOM de l'image tomographique des trois coupes transversales orthogonales et de l'image oblique.

9. Appareil destiné à commander une formation d'images de tomosynthèse selon la revendication 1, dans lequel, lors de la désignation de l'image oblique affichée, une image oblique basée sur au moins une coupe transversale passant par une région tridimensionnelle dans l'image tomographique, identifiée conformément à une opération d'entrée à partir d'un moyen d'exploitation, est désignée, et
dans lequel, lors de la délivrance de l'image DICOM, des données de volume partiel de la région tridimensionnelle sont délivrées en sortie en plus de l'image DICOM de l'image oblique désignée.

10. Appareil destiné à commander une formation d'images de tomosynthèse selon la revendication 1, dans lequel l'au moins un processeur (501) est en outre configuré pour :
générer des données d'image DICOM comprenant l'image oblique désignée en tant que données d'image, et comprenant les informations concernant la condition de formation d'images définie et les informations permettant d'identifier la position et l'orientation de l'image oblique, en tant qu'informations associées.

11. Appareil destiné à commander une formation d'images de tomosynthèse selon la revendication 10, dans lequel, lors de la génération de données d'image DICOM, une image DICOM de l'image oblique désignée, comprenant des premières informations d'identification en tant qu'informations associées, est générée, et une image DICOM de l'image tomographique de trois coupes transversales orthogonales, comprenant des secondes informations d'identification différentes des premières informations d'identification en tant qu'informations associées, est générée.

12. Appareil de gestion d'images (114, 115, 116, 117) destiné à recevoir une image DICOM délivrée en sortie à partir de l'appareil (101, 107) destiné à commander une formation d'images de tomosynthèse selon la revendication 11, l'appareil de gestion d'image comprenant :
au moins un processeur ; et
au moins une mémoire comprenant un programme comprenant des instructions à exécuter par l'au moins un processeur pour mettre en oeuvre un traitement, l'au moins un processeur étant configuré pour :
effectuer un paramétrage de différenciation d'une période de mémorisation ou d'un format d'image de mémorisation entre l'image DICOM devant recevoir les premières informations d'identification et l'image DICOM devant recevoir les secondes informations d'identification.

13. Procédé destiné à commander une formation d'images de tomosynthèse au moyen d'un dispositif de formation d'images comprenant un capteur (106) dans lequel une pluralité de pixels sont disposés de manière bidimensionnelle, le procédé consistant à :
définir une condition de formation d'images de formation d'images de tomosynthèse ;
transmettre un signal, au dispositif de formation d'images, pour commander le capteur (106) sur la base de la condition de formation d'images définie ;
amener un dispositif d'affichage (109) à afficher une image tomographique, comprenant une image oblique, sur la base d'une pluralité d'images de projection obtenues par le capteur (106) ;
désigner l'image oblique ;
obtenir des informations concernant une position et une orientation de l'image oblique désignée ; et
délivrer en sortie une image DICOM comprenant l'image oblique désignée en tant qu'image obtenue lors de la formation d'images de tomosynthèse, et comprenant des informations concernant la condition de formation d'images définie et des informations permettant d'identifier la position et l'orientation de l'image oblique, dans des informations associées.

14. Support d'enregistrement lisible par ordinateur contenant en mémoire un programme qui, lorsqu'il est exécuté sur un ordinateur, amène l'ordinateur à mettre en oeuvre le procédé de commande selon la revendication 13.
